# EUROPEAN PATENT APPLICATION

(11) **EP 2 213 686 A1**
(43) Date of publication of application: **04.08.2010**
(21) Application number: 09151561.9
(22) Date of filing: 28.01.2009
(51) Int. Cl.: C07K 16/30, C12Q 1/68, G01N 33/574

(54) **Tumor markers and methods of use thereof**

(71) Applicant: Externautics S.p.A., 53100 Siena (IT)
(72) Inventor: Grifantini, Renata, 53100 Siena (IT); Pileri, Piero, 53100 Siena (IT); Campagnoli, Susanna, 53100 Siena (IT); Pierleoni, Andrea, 53100 Siena (IT); Nogarotto, Renzo, 53100 Siena (IT)
(74) Representative: Banfi, Paolo

(57) **Abstract**

The invention provides newly identified proteins as markers for the detection of tumors, or as targets for their treatment, particularly of tumors affecting lung, colon, breast, ovary; affinity ligands capable of selectively interacting with the newly identified markers; methods of screening a tissue sample for malignancy, for determining the presence of a tumor in a subject and for screening a test compound as an antitumor candidate; a diagnostic kit.

SEQUENCE LISTING

<110>  Externautics S.p.A.

<120>  TUMOR MARKERS AND METHOS OF USE THEREOF

<130>  2146EUR

<160>  36

<170>  PatentIn version 3.3

<210>  1
<211>  223
<212>  PRT
<213>  Homo sapiens

<400>  1

Met Arg Gly Asn Leu Ala Leu Val Gly Val Leu Ile Ser Leu Ala Phe
1               5                   10                  15

Leu Ser Leu Leu Pro Ser Gly His Pro Gln Pro Ala Gly Asp Asp Ala
            20                  25                  30

Cys Ser Val Gln Ile Leu Val Pro Gly Leu Lys Gly Glu Lys Gly Asp
        35                  40                  45

Ser Gly Asp Ile Gly Pro Pro Gly Pro Asn Gly Glu Pro Gly Leu Pro
    50                  55                  60

Cys Glu Cys Ser Gln Leu Arg Lys Ala Ile Gly Glu Met Asp Asn Gln
65                  70                  75                  80

Val Ser Gln Leu Thr Ser Glu Leu Lys Phe Ile Lys Asn Ala Val Ala
                85                  90                  95

Gly Val Arg Glu Thr Glu Ser Lys Ile Tyr Leu Leu Val Lys Glu Glu
            100                 105                 110

Lys Arg Tyr Ala Asp Ala Gln Leu Ser Cys Gln Gly Arg Gly Gly Thr
        115                 120                 125

Leu Ser Met Pro Lys Asp Glu Ala Ala Asn Gly Leu Met Ala Ala Tyr
    130                 135                 140

Leu Ala Gln Ala Gly Leu Ala Arg Val Phe Ile Gly Ile Asn Asp Leu
145                 150                 155                 160

Glu Lys Glu Gly Ala Phe Val Tyr Ser Asp His Ser Pro Met Arg Thr
                165                 170                 175

Phe Asn Lys Trp Arg Ser Gly Glu Pro Asn Asn Ala Tyr Asp Glu Glu
            180                 185                 190

Asp Cys Val Glu Met Val Ala Ser Gly Gly Trp Asn Asp Val Ala Cys
        195                 200                 205

His Thr Thr Met Tyr Phe Met Cys Glu Phe Asp Lys Glu Asn Met
    210                 215                 220

<210>  2
<211>  247
<212>  PRT
<213>  Homo sapiens

<400>  2

Met Arg Gly Asn Leu Ala Leu Val Gly Val Leu Ile Ser Leu Ala Phe
1               5                   10                  15

Leu Ser Leu Leu Pro Ser Gly His Pro Gln Pro Ala Gly Asp Asp Ala
            20                  25                  30

Cys Ser Val Gln Ile Leu Val Pro Gly Leu Lys Gly Asp Met Gly Asp
        35                  40                  45

Lys Gly Gln Lys Gly Ser Val Gly Arg His Gly Lys Ile Gly Pro Ile
    50                  55                  60

Gly Ser Lys Gly Glu Lys Gly Asp Ser Gly Asp Ile Gly Pro Pro Gly
65                  70                  75                  80

Pro Asn Gly Glu Pro Gly Leu Pro Cys Glu Cys Ser Gln Leu Arg Lys
                85                  90                  95

Ala Ile Gly Glu Met Asp Asn Gln Val Ser Gln Leu Thr Ser Glu Leu
            100                 105                 110

Lys Phe Ile Lys Asn Ala Val Ala Gly Val Arg Glu Thr Glu Ser Lys
        115                 120                 125

Ile Tyr Leu Leu Val Lys Glu Glu Lys Arg Tyr Ala Asp Ala Gln Leu
    130                 135                 140

Ser Cys Gln Gly Arg Gly Gly Thr Leu Ser Met Pro Lys Asp Glu Ala
145                 150                 155                 160

Ala Asn Gly Leu Met Ala Ala Tyr Leu Ala Gln Ala Gly Leu Ala Arg
                165                 170                 175

Val Phe Ile Gly Ile Asn Asp Leu Glu Lys Glu Gly Ala Phe Val Tyr
            180                 185                 190

Ser Asp His Ser Pro Met Arg Thr Phe Asn Lys Trp Arg Ser Gly Glu
        195                 200                 205

Pro Asn Asn Ala Tyr Asp Glu Glu Asp Cys Val Glu Met Val Ala Ser
    210                 215                 220

Gly Gly Trp Asn Asp Val Ala Cys His Thr Thr Met Tyr Phe Met Cys
225                 230                 235                 240

Glu Phe Asp Lys Glu Asn Met
                245

<210>  3
<211>  116
<212>  PRT
<213>  Homo sapiens

<400>  3

Met Arg Gly Asn Leu Ala Leu Val Gly Val Leu Ile Ser Leu Ala Phe
1               5                   10                  15

Leu Ser Leu Leu Pro Ser Gly His Pro Gln Pro Ala Gly Asp Asp Ala
            20                  25                  30

Cys Ser Val Gln Ile Leu Val Pro Gly Leu Lys Gly Asp Ala Gly Glu
        35                  40                  45

Lys Gly Asp Lys Gly Ala Pro Gly Arg Pro Gly Arg Val Gly Pro Thr
    50                  55                  60

Gly Glu Lys Gly Asp Met Gly Asp Lys Gly Gln Lys Gly Ser Val Gly
65                  70                  75                  80

Arg His Gly Lys Ile Gly Pro Ile Gly Ser Lys Gly Glu Lys Gly Asp
                85                  90                  95

Ser Gly Asp Ile Gly Pro Pro Gly Pro Asn Gly Glu Pro Ala Pro Ser
            100                 105                 110

Ala Pro Gln Arg
        115

<210>  4
<211>  43
<212>  PRT

<213>  Homo sapiens

<400>  4

Met Gly Glu Lys Val Pro Glu Ala Ala Val Ser Glu Ala Ser Arg Ala
1               5                   10                  15

Ala Ser Ser Arg Asp Ala Gly Glu Lys Gly Asp Lys Gly Ala Pro Gly
            20                  25                  30

Arg Pro Gly Arg Val Gly Pro Thr Gly Glu Lys
        35                  40

<210>  5
<211>  123
<212>  PRT
<213>  Homo sapiens

<400>  5

Met Lys Lys Gln Arg Gly Val Gly Val Leu Pro Ala Leu Arg Met Arg
1               5                   10                  15

Gly Asn Leu Ala Leu Val Gly Val Leu Ile Ser Leu Ala Phe Leu Ser
            20                  25                  30

Leu Leu Pro Ser Gly His Pro Gln Pro Ala Gly Asp Asp Ala Cys Ser
        35                  40                  45

Val Gln Ile Leu Val Pro Gly Leu Lys Gly Asp Ala Gly Glu Lys Gly
    50                  55                  60

Asp Lys Gly Ala Pro Gly Arg Pro Gly Arg Val Gly Pro Thr Gly Glu
65                  70                  75                  80

Lys Gly Asp Met Gly Asp Lys Gly Gln Lys Gly Ser Val Gly Arg His
                85                  90                  95

Gly Lys Ile Gly Pro Ile Gly Ser Lys Gly Glu Lys Gly Asp Ser Gly
            100                 105                 110

Asp Ile Gly Pro Pro Gly Pro Asn Gly Glu Pro
        115                 120

<210>  6
<211>  221
<212>  PRT
<213>  Homo sapiens

<400>  6

Met Trp Trp Val Pro Pro Ser Pro Tyr Gly Cys Leu Pro Cys Ala Leu
1               5                   10                  15

Pro Gly Asp Ala Gly Glu Lys Gly Asp Lys Gly Ala Pro Gly Arg Pro
            20                  25                  30

Gly Arg Val Gly Pro Thr Gly Glu Lys Gly Glu Lys Gly Asp Ser Gly
        35                  40                  45

Asp Ile Gly Pro Pro Gly Pro Asn Gly Glu Pro Gly Leu Pro Cys Glu
    50                  55                  60

Cys Ser Gln Leu Arg Lys Ala Ile Gly Glu Met Asp Asn Gln Val Ser
65                  70                  75                  80

Gln Leu Thr Ser Glu Leu Lys Phe Ile Lys Asn Ala Val Ala Gly Val
                85                  90                  95

Arg Glu Thr Glu Ser Lys Ile Tyr Leu Leu Val Lys Glu Glu Lys Arg
            100                 105                 110

Tyr Ala Asp Ala Gln Leu Ser Cys Gln Gly Arg Gly Gly Thr Leu Ser
        115                 120                 125

Met Pro Lys Asp Glu Ala Ala Asn Gly Leu Met Ala Ala Tyr Leu Ala
    130                 135                 140

Gln Ala Gly Leu Ala Arg Val Phe Ile Gly Ile Asn Asp Leu Glu Lys
145                 150                 155                 160

Glu Gly Ala Phe Val Tyr Ser Asp His Ser Pro Met Arg Thr Phe Asn
                165                 170                 175

Lys Trp Arg Ser Gly Glu Pro Asn Asn Ala Tyr Asp Glu Glu Asp Cys
            180                 185                 190

Val Glu Met Val Ala Ser Gly Gly Trp Asn Asp Val Ala Cys His Thr
        195                 200                 205

Thr Met Tyr Phe Met Cys Glu Phe Asp Lys Glu Asn Met
    210                 215                 220

<210>  7
<211>  245
<212>  PRT
<213>  Homo sapiens

<400>  7

Met Trp Trp Val Pro Pro Ser Pro Tyr Gly Cys Leu Pro Cys Ala Leu
1               5                   10                  15

Pro Gly Asp Ala Gly Glu Lys Gly Asp Lys Gly Ala Pro Gly Arg Pro
            20                  25                  30

Gly Arg Val Gly Pro Thr Gly Glu Lys Gly Asp Met Gly Asp Lys Gly
        35                  40                  45

Gln Lys Gly Ser Val Gly Arg His Gly Lys Ile Gly Pro Ile Gly Ser
    50                  55                  60

Lys Gly Glu Lys Gly Asp Ser Gly Asp Ile Gly Pro Pro Gly Pro Asn
65                  70                  75                  80

Gly Glu Pro Gly Leu Pro Cys Glu Cys Ser Gln Leu Arg Lys Ala Ile
                85                  90                  95

Gly Glu Met Asp Asn Gln Val Ser Gln Leu Thr Ser Glu Leu Lys Phe
            100                 105                 110

Ile Lys Asn Ala Val Ala Gly Val Arg Glu Thr Glu Ser Lys Ile Tyr
        115                 120                 125

Leu Leu Val Lys Glu Glu Lys Arg Tyr Ala Asp Ala Gln Leu Ser Cys
    130                 135                 140

Gln Gly Arg Gly Gly Thr Leu Ser Met Pro Lys Asp Glu Ala Ala Asn
145                 150                 155                 160

Gly Leu Met Ala Ala Tyr Leu Ala Gln Ala Gly Leu Ala Arg Val Phe
                165                 170                 175

Ile Gly Ile Asn Asp Leu Glu Lys Glu Gly Ala Phe Val Tyr Ser Asp
            180                 185                 190

His Ser Pro Met Arg Thr Phe Asn Lys Trp Arg Ser Gly Glu Pro Asn
        195                 200                 205

Asn Ala Tyr Asp Glu Glu Asp Cys Val Glu Met Val Ala Ser Gly Gly
    210                 215                 220

Trp Asn Asp Val Ala Cys His Thr Thr Met Tyr Phe Met Cys Glu Phe
225                 230                 235                 240

Asp Lys Glu Asn Met
                245

<210>  8
<211>  197
<212>  PRT
<213>  Homo sapiens

<400>  8

Met Trp Trp Val Pro Pro Ser Pro Tyr Gly Cys Leu Pro Cys Ala Leu
1               5                   10                  15

Pro Gly Glu Lys Gly Asp Ser Gly Asp Ile Gly Pro Pro Gly Pro Asn
            20                  25                  30

Gly Glu Pro Gly Leu Pro Cys Glu Cys Ser Gln Leu Arg Lys Ala Ile
        35                  40                  45

Gly Glu Met Asp Asn Gln Val Ser Gln Leu Thr Ser Glu Leu Lys Phe
    50                  55                  60

Ile Lys Asn Ala Val Ala Gly Val Arg Glu Thr Glu Ser Lys Ile Tyr
65                  70                  75                  80

Leu Leu Val Lys Glu Glu Lys Arg Tyr Ala Asp Ala Gln Leu Ser Cys
                85                  90                  95

Gln Gly Arg Gly Gly Thr Leu Ser Met Pro Lys Asp Glu Ala Ala Asn
            100                 105                 110

Gly Leu Met Ala Ala Tyr Leu Ala Gln Ala Gly Leu Ala Arg Val Phe
        115                 120                 125

Ile Gly Ile Asn Asp Leu Glu Lys Glu Gly Ala Phe Val Tyr Ser Asp
    130                 135                 140

His Ser Pro Met Arg Thr Phe Asn Lys Trp Arg Ser Gly Glu Pro Asn
145                 150                 155                 160

Asn Ala Tyr Asp Glu Glu Asp Cys Val Glu Met Val Ala Ser Gly Gly
                165                 170                 175

Trp Asn Asp Val Ala Cys His Thr Thr Met Tyr Phe Met Cys Glu Phe
            180                 185                 190

Asp Lys Glu Asn Met
        195


<210>  9
<211>  221
<212>  PRT

<213>  Homo sapiens

<400>  9

Met Trp Trp Val Pro Pro Ser Pro Tyr Gly Cys Leu Pro Cys Ala Leu
1               5                   10                  15

Pro Gly Asp Met Gly Asp Lys Gly Gln Lys Gly Ser Val Gly Arg His
            20                  25                  30

Gly Lys Ile Gly Pro Ile Gly Ser Lys Gly Glu Lys Gly Asp Ser Gly
        35                  40                  45

Asp Ile Gly Pro Pro Gly Pro Asn Gly Glu Pro Gly Leu Pro Cys Glu
    50                  55                  60

Cys Ser Gln Leu Arg Lys Ala Ile Gly Glu Met Asp Asn Gln Val Ser
65                  70                  75                  80

Gln Leu Thr Ser Glu Leu Lys Phe Ile Lys Asn Ala Val Ala Gly Val
                85                  90                  95

Arg Glu Thr Glu Ser Lys Ile Tyr Leu Leu Val Lys Glu Glu Lys Arg
            100                 105                 110

Tyr Ala Asp Ala Gln Leu Ser Cys Gln Gly Arg Gly Gly Thr Leu Ser
        115                 120                 125

Met Pro Lys Asp Glu Ala Ala Asn Gly Leu Met Ala Ala Tyr Leu Ala
    130                 135                 140

Gln Ala Gly Leu Ala Arg Val Phe Ile Gly Ile Asn Asp Leu Glu Lys
145                 150                 155                 160

Glu Gly Ala Phe Val Tyr Ser Asp His Ser Pro Met Arg Thr Phe Asn
                165                 170                 175

Lys Trp Arg Ser Gly Glu Pro Asn Asn Ala Tyr Asp Glu Glu Asp Cys
            180                 185                 190

Val Glu Met Val Ala Ser Gly Gly Trp Asn Asp Val Ala Cys His Thr
        195                 200                 205

Thr Met Tyr Phe Met Cys Glu Phe Asp Lys Glu Asn Met
    210                 215                 220

<210>  10
<211>  271

<212>  PRT
<213>  Homo sapiens

<400>  10

<210>  11
<211>  268
<212>  PRT
<213>  Homo sapiens

<400>  11

Met Thr Pro Ala Leu Cys Arg Ser Ser Ser Leu Ala Ser Lys Gly Met
1               5                   10                  15

Arg Glu Arg Arg Glu Thr Lys Ala Pro Pro Asp Gly Leu Glu Glu Ser
            20                  25                  30

Ala Pro Arg Glu Lys Lys Gln Ser Gln Pro Val Val Thr Ala Ser Asp
        35                  40                  45

Ile Ser Lys Arg Lys Cys Thr Ser Ser Phe Val Glu Met Gly Ser Gln
    50                  55                  60

Gly Asp Met Gly Asp Lys Gly Gln Lys Gly Ser Val Gly Arg His Gly
65                  70                  75                  80

Lys Ile Gly Pro Ile Gly Ser Lys Gly Glu Lys Gly Asp Ser Gly Asp
                85                  90                  95

Ile Gly Pro Pro Gly Pro Asn Gly Glu Pro Gly Leu Pro Cys Glu Cys
            100                 105                 110

Ser Gln Leu Arg Lys Ala Ile Gly Glu Met Asp Asn Gln Val Ser Gln
        115                 120                 125

Leu Thr Ser Glu Leu Lys Phe Ile Lys Asn Ala Val Ala Gly Val Arg
    130                 135                 140

Glu Thr Glu Ser Lys Ile Tyr Leu Leu Val Lys Glu Glu Lys Arg Tyr
145                 150                 155                 160

Ala Asp Ala Gln Leu Ser Cys Gln Gly Arg Gly Gly Thr Leu Ser Met
                165                 170                 175

Pro Lys Asp Glu Ala Ala Asn Gly Leu Met Ala Ala Tyr Leu Ala Gln
            180                 185                 190

Ala Gly Leu Ala Arg Val Phe Ile Gly Ile Asn Asp Leu Glu Lys Glu
        195                 200                 205

Gly Ala Phe Val Tyr Ser Asp His Ser Pro Met Arg Thr Phe Asn Lys
    210                 215                 220

Trp Arg Ser Gly Glu Pro Asn Asn Ala Tyr Asp Glu Glu Asp Cys Val
225                 230                 235                 240

Glu Met Val Ala Ser Gly Gly Trp Asn Asp Val Ala Cys His Thr Thr
            245                 250                 255

Met Tyr Phe Met Cys Glu Phe Asp Lys Glu Asn Met
            260                 265

<210>  12
<211>  247
<212>  PRT
<213>  Homo sapiens

<400>  12

Met Arg Gly Asn Leu Ala Leu Val Gly Val Leu Ile Ser Leu Ala Phe
1               5                   10                  15

Leu Ser Leu Leu Pro Ser Gly His Pro Gln Pro Ala Gly Asp Asp Ala
            20                  25                  30

Cys Ser Val Gln Ile Leu Val Pro Gly Leu Lys Gly Asp Ala Gly Glu
        35                  40                  45

Lys Gly Asp Lys Gly Ala Pro Gly Arg Pro Gly Arg Val Gly Pro Thr
    50                  55                  60

Gly Glu Lys Gly Glu Lys Gly Asp Ser Gly Asp Ile Gly Pro Pro Gly
65                  70                  75                  80

Pro Asn Gly Glu Pro Gly Leu Pro Cys Glu Cys Ser Gln Leu Arg Lys
                85                  90                  95

Ala Ile Gly Glu Met Asp Asn Gln Val Ser Gln Leu Thr Ser Glu Leu
            100                 105                 110

Lys Phe Ile Lys Asn Ala Val Ala Gly Val Arg Glu Thr Glu Ser Lys
        115                 120                 125

Ile Tyr Leu Leu Val Lys Glu Glu Lys Arg Tyr Ala Asp Ala Gln Leu
    130                 135                 140

Ser Cys Gln Gly Arg Gly Gly Thr Leu Ser Met Pro Lys Asp Glu Ala
145                 150                 155                 160

Ala Asn Gly Leu Met Ala Ala Tyr Leu Ala Gln Ala Gly Leu Ala Arg
                165                 170                 175

Val Phe Ile Gly Ile Asn Asp Leu Glu Lys Glu Gly Ala Phe Val Tyr
            180                 185                 190

Ser Asp His Ser Pro Met Arg Thr Phe Asn Lys Trp Arg Ser Gly Glu
        195                 200                 205

Pro Asn Asn Ala Tyr Asp Glu Glu Asp Cys Val Glu Met Val Ala Ser
    210                 215                 220

Gly Gly Trp Asn Asp Val Ala Cys His Thr Thr Met Tyr Phe Met Cys
225                 230                 235                 240

Glu Phe Asp Lys Glu Asn Met
                245

<210>  13
<211>  849
<212>  PRT
<213>  Homo sapiens

<400>  13

Met Phe Lys Cys Trp Ser Val Val Leu Val Leu Gly Phe Ile Phe Leu
1               5                   10                  15

Glu Ser Glu Gly Arg Pro Thr Lys Glu Gly Gly Tyr Gly Leu Lys Ser
            20                  25                  30

Tyr Gln Pro Leu Met Arg Leu Arg His Lys Gln Glu Lys Asn Gln Glu
        35                  40                  45

Ser Ser Arg Val Lys Gly Phe Met Ile Gln Asp Gly Pro Phe Gly Ser
    50                  55                  60

Cys Glu Asn Lys Tyr Cys Gly Leu Gly Arg His Cys Val Thr Ser Arg
65                  70                  75                  80

Glu Thr Gly Gln Ala Glu Cys Ala Cys Met Asp Leu Cys Lys Arg His
                85                  90                  95

Tyr Lys Pro Val Cys Gly Ser Asp Gly Glu Phe Tyr Glu Asn His Cys
            100                 105                 110

Glu Val His Arg Ala Ala Cys Leu Lys Lys Gln Lys Ile Thr Ile Val
        115                 120                 125

His Asn Glu Asp Cys Phe Phe Lys Gly Asp Lys Cys Lys Thr Thr Glu
    130                 135                 140

Tyr Ser Lys Met Lys Asn Met Leu Leu Asp Leu Gln Asn Gln Lys Tyr
145                 150                 155                 160

Ile Met Gln Glu Asn Glu Asn Pro Asn Gly Asp Asp Ile Ser Arg Lys
                165                 170                 175

Lys Leu Leu Val Asp Gln Met Phe Lys Tyr Phe Asp Ala Asp Ser Asn
            180                 185                 190

Gly Leu Val Asp Ile Asn Glu Leu Thr Gln Val Ile Lys Gln Glu Glu
        195                 200                 205

Leu Gly Lys Asp Leu Phe Asp Cys Thr Leu Tyr Val Leu Leu Lys Tyr
    210                 215                 220

Asp Asp Phe Asn Ala Asp Lys His Leu Ala Leu Glu Glu Phe Tyr Arg
225                 230                 235                 240

Ala Phe Gln Val Ile Gln Leu Ser Leu Pro Glu Asp Gln Lys Leu Ser
                245                 250                 255

Ile Thr Ala Ala Thr Val Gly Gln Ser Ala Val Leu Ser Cys Ala Ile
            260                 265                 270

Gln Gly Thr Leu Arg Pro Pro Ile Ile Trp Lys Arg Asn Asn Ile Ile
        275                 280                 285

Leu Asn Asn Leu Asp Leu Glu Asp Ile Asn Asp Phe Gly Asp Asp Gly
    290                 295                 300

Ser Leu Tyr Ile Thr Lys Val Thr Thr Thr His Val Gly Asn Tyr Thr
305                 310                 315                 320

Cys Tyr Ala Asp Gly Tyr Glu Gln Val Tyr Gln Thr His Ile Phe Gln
                325                 330                 335

Val Asn Val Pro Pro Val Ile Arg Val Tyr Pro Glu Ser Gln Ala Arg
            340                 345                 350

Glu Pro Gly Val Thr Ala Ser Leu Arg Cys His Ala Glu Gly Ile Pro
            355                 360                 365

Lys Pro Gln Leu Gly Trp Leu Lys Asn Gly Ile Asp Ile Thr Pro Lys
        370                 375                 380

Leu Ser Lys Gln Leu Thr Leu Gln Ala Asn Gly Ser Glu Val His Ile
385                 390                 395                 400

Ser Asn Val Arg Tyr Glu Asp Thr Gly Ala Tyr Thr Cys Ile Ala Lys
                405                 410                 415

Asn Glu Ala Gly Val Asp Glu Asp Ile Ser Ser Leu Phe Val Glu Asp
            420                 425                 430

Ser Ala Arg Lys Thr Leu Ala Asn Ile Leu Trp Arg Glu Glu Gly Leu
            435                 440                 445

Gly Ile Gly Asn Met Phe Tyr Val Phe Tyr Glu Asp Gly Ile Lys Val
    450                 455                 460

Ile Gln Pro Ile Glu Cys Glu Phe Gln Arg His Ile Lys Pro Ser Glu
465                 470                 475                 480

Lys Leu Leu Gly Phe Gln Ala Lys Asp Glu Val Cys Pro Lys Ala Glu
                485                 490                 495

Gly Asp Glu Val Gln Arg Cys Val Trp Ala Ser Ala Val Asn Val Lys
            500                 505                 510

Asp Lys Phe Ile Tyr Val Ala Gln Pro Thr Leu Asp Arg Val Leu Ile
            515                 520                 525

Val Asp Val Gln Ser Gln Lys Val Val Gln Ala Val Ser Thr Asp Pro
    530                 535                 540

Val Pro Val Lys Leu His Tyr Asp Lys Ser His Asp Gln Val Trp Val
545                 550                 555                 560

Leu Ser Trp Gly Thr Leu Glu Lys Thr Ser Pro Thr Leu Gln Val Ile
                565                 570                 575

Thr Leu Ala Ser Gly Asn Val Pro His His Thr Ile His Thr Gln Pro
            580                 585                 590

Val Gly Lys Gln Phe Asp Arg Val Asp Asp Phe Phe Ile Pro Thr Thr
            595                 600                 605

Thr Leu Ile Ile Thr His Met Arg Phe Gly Phe Ile Leu His Lys Asp
    610                 615                 620

Glu Ala Ala Leu Gln Lys Ile Asp Leu Glu Thr Met Ser Tyr Ile Lys
625                 630                 635                 640

Thr Ile Asn Leu Lys Asp Tyr Lys Cys Val Pro Gln Ser Leu Ala Tyr
                645                 650                 655

Thr His Leu Gly Gly Tyr Tyr Phe Ile Gly Cys Lys Pro Asp Ser Thr
            660                 665                 670

Gly Ala Val Ser Pro Gln Val Met Val Asp Gly Val Thr Asp Ser Val
        675                 680                 685

Ile Gly Phe Asn Ser Asp Val Thr Gly Thr Pro Tyr Val Ser Pro Asp
    690                 695                 700

Gly His Tyr Leu Val Ser Ile Asn Asp Val Lys Gly Leu Val Arg Val
705                 710                 715                 720

Gln Tyr Ile Thr Ile Arg Gly Glu Ile Gln Glu Ala Phe Asp Ile Tyr
                725                 730                 735

Thr Asn Leu His Ile Ser Asp Leu Ala Phe Gln Pro Ser Phe Thr Glu
            740                 745                 750

Ala His Gln Tyr Asn Ile Tyr Gly Ser Ser Ser Thr Gln Thr Asp Val
        755                 760                 765

Leu Phe Val Glu Leu Ser Ser Gly Lys Val Lys Met Ile Lys Ser Leu
    770                 775                 780

Lys Glu Pro Leu Lys Ala Glu Glu Trp Pro Trp Asn Arg Lys Asn Arg
785                 790                 795                 800

Gln Ile Gln Asp Ser Gly Leu Phe Gly Gln Tyr Leu Met Thr Pro Ser
                805                 810                 815

Lys Asp Ser Leu Phe Ile Leu Asp Gly Arg Leu Asn Lys Leu Asn Cys
            820                 825                 830

Glu Ile Thr Glu Val Glu Lys Gly Asn Thr Val Ile Trp Val Gly Asp
        835                 840                 845

Ala

<210>  14
<211>  847
<212>  PRT
<213>  Homo sapiens

<400>  14

Met Phe Lys Cys Trp Ser Val Val Leu Val Leu Gly Phe Ile Phe Leu
1               5                   10                  15

Glu Ser Glu Gly Arg Pro Thr Lys Glu Gly Gly Tyr Gly Leu Lys Ser
            20                  25                  30

Tyr Gln Pro Leu Met Arg Leu Arg His Lys Gln Glu Lys Asn Gln Glu
        35                  40                  45

Ser Ser Arg Val Lys Gly Phe Met Ile Gln Asp Gly Pro Phe Gly Ser
    50                  55                  60

Cys Glu Asn Lys Tyr Cys Gly Leu Gly Arg His Cys Val Thr Ser Arg
65                  70                  75                  80

Glu Thr Gly Gln Ala Glu Cys Ala Cys Met Asp Leu Cys Lys Arg His
                85                  90                  95

Tyr Lys Pro Val Cys Gly Ser Asp Gly Glu Phe Tyr Glu Asn His Cys
            100                 105                 110

Glu Val His Arg Ala Ala Cys Leu Lys Lys Gln Lys Ile Thr Ile Val
        115                 120                 125

His Asn Glu Asp Cys Phe Phe Lys Gly Asp Lys Cys Lys Thr Thr Glu
    130                 135                 140

Tyr Ser Lys Met Lys Asn Met Leu Leu Asp Leu Gln Asn Gln Lys Tyr
145                 150                 155                 160

Ile Met Gln Glu Asn Glu Asn Pro Asn Gly Asp Asp Ile Ser Arg Lys
                165                 170                 175

Lys Leu Leu Val Asp Gln Met Phe Lys Tyr Phe Asp Ala Asp Ser Asn
            180                 185                 190

Gly Leu Val Asp Ile Asn Glu Leu Thr Gln Val Ile Lys Gln Glu Glu
        195                 200                 205

Leu Gly Lys Asp Leu Phe Asp Cys Thr Leu Tyr Val Leu Leu Lys Tyr
    210                 215                 220

Asp Asp Phe Asn Ala Asp Lys His Leu Ala Leu Glu Glu Phe Tyr Arg
225                 230                 235                 240

Ala Phe Gln Val Ile Gln Leu Ser Leu Pro Glu Asp Gln Lys Leu Ser
                245                 250                 255

Ile Thr Ala Ala Thr Val Gly Gln Ser Ala Val Leu Ser Cys Ala Ile
            260                 265                 270

Gln Gly Thr Leu Arg Pro Pro Ile Ile Trp Lys Arg Asn Asn Ile Ile
        275                 280                 285

Leu Asn Asn Leu Asp Leu Glu Asp Ile Asn Asp Phe Gly Asp Asp Gly
    290                 295                 300

Ser Leu Tyr Ile Thr Lys Val Thr Thr Thr His Val Gly Asn Tyr Thr
305                 310                 315                 320

Cys Tyr Ala Asp Gly Tyr Glu Gln Val Tyr Gln Thr His Ile Phe Gln
                325                 330                 335

Val Asn Val Pro Pro Val Ile Arg Val Tyr Pro Glu Ser Gln Ala Arg
            340                 345                 350

Glu Pro Gly Val Thr Ala Ser Leu Arg Cys His Ala Glu Gly Ile Pro
        355                 360                 365

Lys Pro Gln Leu Gly Trp Leu Lys Asn Gly Ile Asp Ile Thr Pro Lys
    370                 375                 380

Leu Ser Lys Gln Leu Thr Leu Gln Ala Asn Gly Ser Glu Val His Ile
385                 390                 395                 400

Ser Asn Val Arg Tyr Glu Asp Thr Gly Ala Tyr Thr Cys Ile Ala Lys
                405                 410                 415

Asn Glu Ala Gly Val Asp Glu Asp Ile Ser Ser Leu Phe Val Glu Asp
            420                 425                 430

Ser Ala Arg Lys Thr Leu Ala Asn Ile Leu Trp Arg Glu Glu Gly Leu
        435                 440                 445

Gly Ile Gly Asn Met Phe Tyr Val Phe Tyr Glu Asp Gly Ile Lys Val
    450                 455                 460

Ile Gln Pro Ile Glu Cys Glu Phe Gln Arg His Ile Lys Pro Ser Glu
465                 470                 475                 480

Lys Leu Leu Gly Phe Gln Asp Glu Val Cys Pro Lys Ala Glu Gly Asp
                485                 490                 495

Glu Val Gln Arg Cys Val Trp Ala Ser Ala Val Asn Val Lys Asp Lys
            500                 505                 510

Phe Ile Tyr Val Ala Gln Pro Thr Leu Asp Arg Val Leu Ile Val Asp
        515                 520                 525

Val Gln Ser Gln Lys Val Val Gln Ala Val Ser Thr Asp Pro Val Pro
    530                 535                 540

Val Lys Leu His Tyr Asp Lys Ser His Asp Gln Val Trp Val Leu Ser
545                 550                 555                 560

Trp Gly Thr Leu Glu Lys Thr Ser Pro Thr Leu Gln Val Ile Thr Leu
                565                 570                 575

Ala Ser Gly Asn Val Pro His His Thr Ile His Thr Gln Pro Val Gly
            580                 585                 590

Lys Gln Phe Asp Arg Val Asp Asp Phe Phe Ile Pro Thr Thr Thr Leu
        595                 600                 605

Ile Ile Thr His Met Arg Phe Gly Phe Ile Leu His Lys Asp Glu Ala
    610                 615                 620

Ala Leu Gln Lys Ile Asp Leu Glu Thr Met Ser Tyr Ile Lys Thr Ile
625                 630                 635                 640

Asn Leu Lys Asp Tyr Lys Cys Val Pro Gln Ser Leu Ala Tyr Thr His
                645                 650                 655

Leu Gly Gly Tyr Tyr Phe Ile Gly Cys Lys Pro Asp Ser Thr Gly Ala
            660                 665                 670

Val Ser Pro Gln Val Met Val Asp Gly Val Thr Asp Ser Val Ile Gly
        675                 680                 685

Phe Asn Ser Asp Val Thr Gly Thr Pro Tyr Val Ser Pro Asp Gly His
    690                 695                 700

Tyr Leu Val Ser Ile Asn Asp Val Lys Gly Leu Val Arg Val Gln Tyr
705                 710                 715                 720

Ile Thr Ile Arg Gly Glu Ile Gln Glu Ala Phe Asp Ile Tyr Thr Asn
                725                 730                 735

Leu His Ile Ser Asp Leu Ala Phe Gln Pro Ser Phe Thr Glu Ala His
            740                 745                 750

Gln Tyr Asn Ile Tyr Gly Ser Ser Ser Thr Gln Thr Asp Val Leu Phe
        755                 760                 765

Val Glu Leu Ser Ser Gly Lys Val Lys Met Ile Lys Ser Leu Lys Glu
    770                 775                 780

Pro Leu Lys Ala Glu Glu Trp Pro Trp Asn Arg Lys Asn Arg Gln Ile
785                 790                 795                 800

Gln Asp Ser Gly Leu Phe Gly Gln Tyr Leu Met Thr Pro Ser Lys Asp
                805                 810                 815

Ser Leu Phe Ile Leu Asp Gly Arg Leu Asn Lys Leu Asn Cys Glu Ile
            820                 825                 830

Thr Glu Val Glu Lys Gly Asn Thr Val Ile Trp Val Gly Asp Ala
        835                 840                 845

<210>  15
<211>  470
<212>  PRT
<213>  Homo sapiens

<400>  15

Met Ser Arg Leu Gly Ala Leu Gly Gly Ala Arg Ala Gly Leu Gly Leu
1               5                   10                  15

Leu Leu Gly Thr Ala Ala Gly Leu Gly Phe Leu Cys Leu Leu Tyr Ser
            20                  25                  30

Gln Arg Trp Lys Arg Thr Gln Arg His Gly Arg Ser Gln Ser Leu Pro
        35                  40                  45

Asn Ser Leu Asp Tyr Thr Gln Thr Ser Asp Pro Gly Arg His Val Met
    50                  55                  60

Leu Leu Arg Ala Val Pro Gly Gly Ala Gly Asp Ala Ser Val Leu Pro
65                  70                  75                  80

Ser Leu Pro Arg Glu Gly Gln Glu Lys Val Leu Asp Arg Leu Asp Phe
                85                  90                  95

Val Leu Thr Ser Leu Val Ala Leu Arg Arg Glu Val Glu Glu Leu Arg
            100                 105                 110

Ser Ser Leu Arg Gly Leu Ala Gly Glu Ile Val Gly Glu Val Arg Cys
        115                 120                 125

His Met Glu Glu Asn Gln Arg Val Ala Arg Arg Arg Arg Phe Pro Phe
    130                 135                 140

Val Arg Glu Arg Ser Asp Ser Thr Gly Ser Ser Ser Val Tyr Phe Thr
145                 150                 155                 160

Ala Ser Ser Gly Ala Thr Phe Thr Asp Ala Glu Ser Glu Gly Gly Tyr
                165                 170                 175

Thr Thr Ala Asn Ala Glu Ser Asp Asn Glu Arg Asp Ser Asp Lys Glu
            180                 185                 190

Ser Glu Asp Gly Glu Asp Glu Val Ser Cys Glu Thr Val Lys Met Gly
        195                 200                 205

Arg Lys Asp Ser Leu Asp Leu Glu Glu Glu Ala Ala Ser Gly Ala Ser
    210                 215                 220

Ser Ala Leu Glu Ala Gly Gly Ser Ser Gly Leu Glu Asp Val Leu Pro
225                 230                 235                 240

Leu Leu Gln Gln Ala Asp Glu Leu His Arg Gly Asp Glu Gln Gly Lys
                245                 250                 255

Arg Glu Gly Phe Gln Leu Leu Leu Asn Asn Lys Leu Val Tyr Gly Ser
            260                 265                 270

Arg Gln Asp Phe Leu Trp Arg Leu Ala Arg Ala Tyr Ser Asp Met Cys
        275                 280                 285

Glu Leu Thr Glu Glu Val Ser Glu Lys Lys Ser Tyr Ala Leu Asp Gly
    290                 295                 300

Lys Glu Glu Ala Glu Ala Ala Leu Glu Lys Gly Asp Glu Ser Ala Asp
305                 310                 315                 320

Cys His Leu Trp Tyr Ala Val Leu Cys Gly Gln Leu Ala Glu His Glu
                325                 330                 335

Ser Ile Gln Arg Arg Ile Gln Ser Gly Phe Ser Phe Lys Glu His Val
            340                 345                 350

Asp Lys Ala Ile Ala Leu Gln Pro Glu Asn Pro Met Ala His Phe Leu
        355                 360                 365

Leu Gly Arg Trp Cys Tyr Gln Val Ser His Leu Ser Trp Leu Glu Lys
    370                 375                 380

Lys Thr Ala Thr Ala Leu Leu Glu Ser Pro Leu Ser Ala Thr Val Glu
385                 390                 395                 400

Asp Ala Leu Gln Ser Phe Leu Lys Ala Glu Glu Leu Gln Pro Gly Phe
                405                 410                 415

Ser Lys Ala Gly Arg Val Tyr Ile Ser Lys Cys Tyr Arg Glu Leu Gly
            420                 425                 430

Lys Asn Ser Glu Ala Arg Trp Trp Met Lys Leu Ala Leu Glu Leu Pro
        435                 440                 445

Asp Val Thr Lys Glu Asp Leu Ala Ile Gln Lys Asp Leu Glu Glu Leu
    450                 455                 460

Glu Val Ile Leu Arg Asp
465                 470

<210>  16
<211>  470
<212>  PRT
<213>  Homo sapiens

<400>  16

Met Ser Arg Leu Gly Ala Leu Gly Gly Ala Arg Ala Gly Leu Gly Leu
1               5                   10                  15

Leu Leu Gly Thr Ala Ala Gly Leu Gly Phe Leu Cys Leu Leu Tyr Ser
            20                  25                  30

Gln Arg Trp Lys Arg Thr Gln Arg His Gly Arg Ser Gln Ser Leu Pro
        35                  40                  45

Asn Ser Leu Asp Tyr Thr Gln Thr Ser Asp Pro Gly Arg His Val Met
    50                  55                  60

Leu Leu Arg Ala Val Pro Gly Gly Ala Gly Asp Ala Ser Val Leu Pro
65                  70                  75                  80

Ser Leu Pro Arg Glu Gly Gln Glu Lys Val Leu Asp Arg Leu Asp Phe
                85                  90                  95

Val Leu Thr Ser Leu Val Ala Leu Arg Arg Glu Val Glu Glu Leu Arg
            100                 105                 110

Ser Ser Leu Arg Gly Leu Ala Gly Glu Ile Val Gly Glu Val Arg Cys
        115                 120                 125

His Met Glu Glu Asn Gln Arg Val Ala Arg Arg Arg Arg Phe Pro Phe
    130                 135                 140

Val Arg Glu Arg Ser Asp Ser Thr Gly Ser Ser Ser Val Tyr Phe Thr
145                 150                 155                 160

Ala Ser Ser Gly Ala Thr Phe Thr Asp Ala Glu Ser Glu Gly Gly Tyr
                165                 170                 175

Thr Thr Ala Asn Ala Glu Ser Asp Asn Glu Arg Asp Ser Asp Lys Glu
            180                 185                 190

Ser Glu Asp Gly Glu Asp Glu Val Ser Cys Glu Thr Val Lys Met Gly
        195                 200                 205

Arg Lys Asp Ser Leu Asp Leu Glu Glu Glu Ala Ala Ser Gly Ala Ser
    210                 215                 220

Ser Ala Leu Glu Ala Gly Gly Ser Ser Gly Leu Glu Asp Val Leu Pro
225                 230                 235                 240

Leu Leu Gln Gln Ala Asp Glu Leu His Arg Gly Asp Glu Gln Gly Lys
                245                 250                 255

Arg Glu Gly Phe Gln Leu Leu Leu Asn Asn Lys Leu Val Tyr Gly Ser
            260                 265                 270

Arg Gln Asp Phe Leu Trp Arg Leu Ala Arg Ala Tyr Ser Asp Met Cys
        275                 280                 285

Glu Leu Thr Glu Glu Val Ser Glu Lys Lys Ser Tyr Ala Leu Asp Gly
    290                 295                 300

Lys Glu Glu Ala Glu Ala Ala Leu Glu Lys Gly Asp Glu Ser Ala Asp
305                 310                 315                 320

Cys His Leu Trp Tyr Ala Val Leu Cys Gly Gln Leu Ala Glu His Glu
                325                 330                 335

Ser Ile Gln Arg Arg Ile Gln Ser Gly Phe Ser Phe Lys Glu His Val
            340                 345                 350

Asp Lys Ala Ile Ala Leu Gln Pro Glu Asn Pro Met Ala His Phe Leu
        355                 360                 365

Leu Gly Arg Trp Cys Tyr Gln Val Ser His Leu Ser Trp Leu Glu Lys
    370                 375                 380

Lys Thr Ala Thr Ala Leu Leu Glu Ser Pro Leu Ser Ala Thr Val Glu
385                 390                 395                 400

Asp Ala Leu Gln Ser Phe Leu Lys Ala Glu Glu Leu Gln Pro Gly Phe
                405                 410                 415

Ser Lys Ala Gly Arg Val Tyr Ile Ser Lys Cys Tyr Arg Glu Leu Gly
            420                 425                 430

Lys Asn Ser Glu Ala Arg Trp Trp Met Lys Leu Ala Leu Glu Leu Pro
        435                 440                 445

Asp Val Thr Lys Glu Asp Leu Ala Ile Gln Lys Asp Leu Glu Glu Leu
    450                 455                 460

Glu Val Ile Leu Arg Asp
465                 470

<210>  17
<211>  276
<212>  PRT
<213>  Homo sapiens

<400>  17

Met Glu Glu Asn Gln Arg Val Ala Arg Arg Arg Arg Phe Pro Phe Val
1               5                   10                  15

Arg Glu Arg Ser Asp Ser Thr Gly Ser Ser Ser Val Tyr Phe Thr Ala
            20                  25                  30

Ser Ser Gly Ala Thr Phe Thr Asp Ala Glu Ser Glu Gly Gly Tyr Thr
        35                  40                  45

Thr Ala Asn Ala Glu Ser Asp Asn Glu Arg Asp Ser Asp Lys Glu Ser
    50                  55                  60

Glu Asp Gly Glu Asp Glu Val Ser Cys Glu Thr Val Lys Met Gly Arg
65                  70                  75                  80

Lys Asp Ser Leu Asp Leu Glu Glu Glu Ala Ala Ser Gly Ala Ser Ser
                85                  90                  95

Ala Leu Glu Ala Gly Gly Ser Ser Gly Leu Glu Asp Val Leu Pro Leu
            100                 105                 110

Leu Gln Gln Ala Asp Glu Leu His Arg Gly Asp Glu Gln Gly Lys Arg
        115                 120                 125

Glu Gly Phe Gln Leu Leu Leu Asn Asn Lys Leu Val Tyr Gly Ser Arg
    130                 135                 140

Gln Asp Phe Leu Trp Arg Leu Ala Arg Ala Tyr Ser Asp Met Cys Glu
145                 150                 155                 160

Leu Thr Glu Glu Val Ser Glu Lys Lys Ser Tyr Ala Leu Asp Gly Lys
                165                 170                 175

Glu Glu Ala Glu Ala Ala Leu Glu Lys Gly Asp Glu Ser Ala Asp Cys
            180                 185                 190

His Leu Trp Tyr Ala Val Leu Cys Gly Gln Leu Ala Glu His Glu Ser
        195                 200                 205

Ile Gln Arg Arg Ile Gln Ser Gly Phe Ser Phe Lys Glu His Val Asp
    210                 215                 220

Lys Ala Ile Ala Leu Gln Pro Glu Asn Pro Met Ala His Phe Leu Leu
225                 230                 235                 240

Gly Arg Trp Cys Tyr Gln Val Ser His Leu Ser Trp Leu Glu Lys Lys
                245                 250                 255

Leu Gln Pro Cys Leu Lys Ala Leu Ser Val Pro Leu Trp Lys Met Pro
            260                 265                 270

Ser Arg Ala Ser
        275

<210>  18
<211>  1378
<212>  DNA
<213>  Homo sapiens

<400>  18
ctagcgcgtg ctcaggagtt ggtgtcctgc ctgcgctcag gatgaggggg aatctggccc      60

tggtgggcgt tctaatcagc ctggccttcc tgtcactgct gccatctgga catcctcagc     120

cggctggcga tgacgcctgc tctgtgcaga tcctcgtccc tggcctcaaa ggggatgcgg     180

gagagaaggg agacaaaggc gcccccggac ggcctggaag agtcggcccc acgggagaaa     240

aaggagacat gggggacaaa ggacagaaag gcagtgtggg tcgtcatgga aaaattggtc     300

ccattggctc taaaggtgag aaaggagatt ccggtgacat aggaccccct ggtcctaatg     360

gagaaccagc tccttcggca ccgcagagat gaggaggaac ggctgcttgg aaggtgtgct 420

tgccggtgtg acttggctga gtgtgagtgc gctgagatca gtgtcactga ctggccgagc 480

agcatccgct cagggcacct ccaggcactc agccaacctg aggggcccgg acaaagcctc 540

tgggagacag aaggacctgg aggcctccca tgtgagtgca gccagctgcg caaggccatc 600

ggggagatgg acaaccaggt ctctcagctg accagcgagc tcaagttcat caagaatgct 660

gtcgccggtg tgcgcgagac ggagagcaag atctacctgc tggtgaagga ggagaagcgc 720

tacgcggacg cccagctgtc ctgccagggc cgcgggggca cgctgagcat gcccaaggac 780

gaggctgcca atggcctgat ggccgcatac ctggcgcaag ccggcctggc ccgtgtcttc 840

atcggcatca acgacctgga gaaggagggc gccttcgtgt actctgacca ctcccccatg 900

cggaccttca acaagtggcg cagcggtgag cccaacaatg cctacgacga ggaggactgc 960

gtggagatgg tggcctcggg cggctggaac gacgtggcct gccacaccac catgtacttc 1020

atgtgtgagt ttgacaagga gaacatgtga gcctcaggct ggggctgccc attgggggcc 1080

ccacatgtcc ctgcagggtt ggcagggaca gagcccagac catggtgcca gccagggagc 1140

tgtccctctg tgaagggtgg aggctcactg agtagagggc tgttgtctaa actgagaaaa 1200

tggcctatgc ttaagaggaa aatgaaagtg ttcctggggt gctgtctctg aagaagcaga 1260

gtttcattac ctgtattgta gccccaatgt cattatgtaa ttattaccca gaattgctct 1320

tccataaagc ttgtgccttt gtccaagcta tacaataaaa tctttaagta gtgcagta 1378

<210> 19
<211> 640
<212> DNA
<213> Homo sapiens

<400> 19
aagaaacaaa attgggatgt tcgggttgga gagttagcag gggcatctcc aggagggtcc 60

tgccccccac cccaaagccg gcctcaggga gttccaggcg ttcccacact ccccaggcag 120

gacggtggac gcagcgcaga caggaagctc cccgagataa cgctgcggcc gggcggcctg 180

atttgctggg ctgtctgatg gcccgggccg aggcttctcc ctgcgcctgg gactgcggcc 240

gcctctctaa atagcagcca tgaggcgcct gggggcagtg tcctcgcggg ccagcgacgg 300

gcaggacgcc ccgttcgcct agcgcgtgct caggagttgg tgtcctgcct gcgctcagga 360

tgagggggaa tctggccctg gtgggcgttc taatcagcct ggccttcctg tcactgctgc 420

catctggaca tcctcagccg gctggcgatg acgcctgctc tgtgcagatc ctcgtccctg 480

gcctcaaaga gatagttctg caggtgaggc atgggtgaga aagtccctga ggctgcggta 540

tctgaagcct ccagagcagc atcttcaagg gatgcgggag agaagggaga caaaggcgcc 600

cccggacggc ctggaagagt cggccccacg ggagaaaaag 640

<210>  20
<211>  581
<212>  DNA
<213>  Homo sapiens

<400>  20
agatttggga gaaggatttc aaatgacttc ccttcgagct gcagggctat cttgggcatc      60

ttgacctcca cacatttctg ggagaactcc tcgtgcagca ggacatcagg tggcaaaggt     120

gatctgccca cctcagcctc ccaaagtgct ggaattacag gtgtgagcca ctgcgcctgg     180

tcttgttttc caagttgtaa cgcccttcac gatgaagaaa caaagaggag ttggtgtcct     240

gcctgcgctc aggatgaggg ggaatctggc cctggtgggc gttctaatca gcctggcctt     300

cctgtcactg ctgccatctg gacatcctca gccggctggc gatgacgcct gctctgtgca     360

gatcctcgtc cctggcctca aaggggatgc gggagagaag ggagacaaag gcgcccccgg     420

acggcctgga agagtcggcc ccacgggaga aaaaggagac atgggggaca aaggacagaa     480

aggcagtgtg ggtcgtcatg gaaaaattgg tcccattggc tctaaaggtg agaaaggaga     540

ttccggtgac ataggacccc ctggtcctaa tggagaacca g                         581


<210>  21
<211>  1134
<212>  DNA
<213>  Homo sapiens

<400>  21
cagaagtttt ggtgaaagtg gctttggccc tgactttgtg gtagcgtgtg tgggtttgtg      60

agtggaacct tcagctttag gttggaaacg gtggctgtgg agagctggac ttttggctgt     120

ggaggtcacg tccctgccca atgtggtggg tgcctccgag tccctacggt tgtcttccct     180

gcgccctgcc aggggatgcg ggagagaagg gagacaaagg cgcccccgga cggcctggaa     240

gagtcggccc cacgggagaa aaaggtgaga aaggagattc cggtgacata ggaccccctg     300

gtcctaatgg agaaccaggc ctcccatgtg agtgcagcca gctgcgcaag gccatcgggg     360

agatggacaa ccaggtctct cagctgacca gcgagctcaa gttcatcaag aatgctgtcg     420

ccggtgtgcg cgagacggag agcaagatct acctgctggt gaaggaggag aagcgctacg     480

cggacgccca gctgtcctgc cagggccgcg ggggcacgct gagcatgccc aaggacgagg     540

ctgccaatgg cctgatggcc gcatacctgg cgcaagccgg cctggcccgt gtcttcatcg     600

gcatcaacga cctggagaag gagggcgcct tcgtgtactc tgaccactcc cccatgcgga     660

ccttcaacaa gtggcgcagc ggtgagccca acaatgccta cgacgaggag gactgcgtgg     720

agatggtggc ctcgggcggc tggaacgacg tggcctgcca caccaccatg tacttcatgt     780

gtgagtttga caaggagaac atgtgagcct caggctgggg ctgcccattg ggggccccac     840

atgtccctgc agggttggca gggacagagc ccagaccatg gtgccagcca gggagctgtc     900

cctctgtgaa gggtggaggc tcactgagta gagggctgtt gtctaaactg agaaaatggc 960

ctatgcttaa gaggaaaatg aaagtgttcc tggggtgctg tctctgaaga agcagagttt 1020

cattacctgt attgtagccc caatgtcatt atgtaattat tacccagaat tgctcttcca 1080

taaagcttgt gcctttgtcc aagctataca ataaaatctt taagtagtgc agta 1134

<210> 22
<211> 1557
<212> DNA
<213> Homo sapiens

<400> 22
tgcggctctt tttattgaaa cattagagaa aagtagattc aaacccatga tcaaaacagt 60

caccacgaaa gacaaaccct gctcagcccg tgttgtacaa aatgaaaagg tcagccctct 120

cccggggcac agggtccgga cagctcgggg ccaccctgcc aggctgtgca gctctggctg 180

gggctttttt tccggctgtg ttagccctgc tgttggaaac caagtggact gtcctgcagc 240

ccagacaagc ccagtggcct ttgtgctttt aattgtgata aatgtacctg ctttagaaaa 300

tgtttaatta tgttggaagc aacttaaact gttggatgcc tacggctctc tcagaagttt 360

tggtgaaagt ggctttggcc ctgactttgt ggtagcgtgt gtgggtttgt gagtggaacc 420

ttcagcttta ggttggaaac ggtggctgtg gagagctgga cttttggctg tggaggtcac 480

gtccctgccc aatgtggtgg gtgcctccga gtccctacgg ttgtcttccc tgcgccctgc 540

caggggatgc gggagagaag ggagacaaag gcgcccccgg acggcctgga agagtcggcc 600

ccacgggaga aaaaggagac atgggggaca aaggacagaa aggcagtgtg ggtcgtcatg 660

gaaaaattgg tcccattggc tctaaaggtg agaaaggaga ttccggtgac ataggacccc 720

ctggtcctaa tggagaacca ggcctcccat gtgagtgcag ccagctgcgc aaggccatcg 780

gggagatgga caaccaggtc tctcagctga ccagcgagct caagttcatc aagaatgctg 840

tcgccggtgt gcgcgagacg gagagcaaga tctacctgct ggtgaaggag gagaagcgct 900

acgcggacgc ccagctgtcc tgccagggcc gcgggggcac gctgagcatg cccaaggacg 960

aggctgccaa tggcctgatg gccgcatacc tggcgcaagc cggcctggcc cgtgtcttca 1020

tcggcatcaa cgacctggag aaggagggcg ccttcgtgta ctctgaccac tcccccatgc 1080

ggaccttcaa caagtggcgc agcggtgagc ccaacaatgc ctacgacgag gaggactgcg 1140

tggagatggt ggcctcgggc ggctggaacg acgtggcctg ccacaccacc atgtacttca 1200

tgtgtgagtt tgacaaggag aacatgtgag cctcaggctg gggctgccca ttgggggccc 1260

cacatgtccc tgcagggttg gcagggacag agcccagacc atggtgccag ccagggagct 1320

gtccctctgt gaagggtgga ggctcactga gtagagggct gttgtctaaa ctgagaaaat 1380

ggcctatgct taagaggaaa atgaaagtgt tcctggggtg ctgtctctga agaagcagag 1440

tttcattacc tgtattgtag ccccaatgtc attatgtaat tattacccag aattgctctt 1500

ccataaagct tgtgcctttg tccaagctat acaataaaat ctttaagtag tgcagta 1557

<210> 23
<211> 1062
<212> DNA
<213> Homo sapiens

<400> 23
cagaagtttt ggtgaaagtg gctttggccc tgactttgtg gtagcgtgtg tgggtttgtg 60

agtggaacct tcagctttag gttggaaacg gtggctgtgg agagctggac ttttggctgt 120

ggaggtcacg tccctgccca atgtggtggg tgcctccgag tccctacggt tgtcttccct 180

gcgccctgcc aggtgagaaa ggagattccg gtgacatagg accccctggt cctaatggag 240

aaccaggcct cccatgtgag tgcagccagc tgcgcaaggc catcggggag atggacaacc 300

aggtctctca gctgaccagc gagctcaagt tcatcaagaa tgctgtcgcc ggtgtgcgcg 360

agacggagag caagatctac ctgctggtga aggaggagaa gcgctacgcg gacgcccagc 420

tgtcctgcca gggccgcggg ggcacgctga gcatgcccaa ggacgaggct gccaatggcc 480

tgatggccgc atacctggcg caagccggcc tggcccgtgt cttcatcggc atcaacgacc 540

tggagaagga gggcgccttc gtgtactctg accactcccc catgcggacc ttcaacaagt 600

ggcgcagcgg tgagcccaac aatgcctacg acgaggagga ctgcgtggag atggtggcct 660

cgggcggctg gaacgacgtg gcctgccaca ccaccatgta cttcatgtgt gagtttgaca 720

aggagaacat gtgagcctca ggctggggct gcccattggg ggccccacat gtccctgcag 780

ggttggcagg gacagagccc agaccatggt gccagccagg gagctgtccc tctgtgaagg 840

gtggaggctc actgagtaga gggctgttgt ctaaactgag aaaatggcct atgcttaaga 900

ggaaaatgaa agtgttcctg gggtgctgtc tctgaagaag cagagtttca ttacctgtat 960

tgtagcccca atgtcattat gtaattatta cccagaattg ctcttccata aagcttgtgc 1020

ctttgtccaa gctatacaat aaaatcttta agtagtgcag ta 1062

<210> 24
<211> 1134
<212> DNA
<213> Homo sapiens

<400> 24
cagaagtttt ggtgaaagtg gctttggccc tgactttgtg gtagcgtgtg tgggtttgtg 60

agtggaacct tcagctttag gttggaaacg gtggctgtgg agagctggac ttttggctgt 120

ggaggtcacg tccctgccca atgtggtggg tgcctccgag tccctacggt tgtcttccct 180

gcgccctgcc aggagacatg ggggacaaag gacagaaagg cagtgtgggt cgtcatggaa 240

aaattggtcc cattggctct aaaggtgaga aaggagattc cggtgacata ggaccccctg 300

gtcctaatgg agaaccaggc ctcccatgtg agtgcagcca gctgcgcaag gccatcgggg 360

agatggacaa ccaggtctct cagctgacca gcgagctcaa gttcatcaag aatgctgtcg 420

ccggtgtgcg cgagacggag agcaagatct acctgctggt gaaggaggag aagcgctacg 480

cggacgccca gctgtcctgc cagggccgcg ggggcacgct gagcatgccc aaggacgagg 540

ctgccaatgg cctgatggcc gcatacctgg cgcaagccgg cctggcccgt gtcttcatcg 600

gcatcaacga cctggagaag gagggcgcct tcgtgtactc tgaccactcc cccatgcgga 660

ccttcaacaa gtggcgcagc ggtgagccca acaatgccta cgacgaggag gactgcgtgg 720

agatggtggc ctcgggcggc tggaacgacg tggcctgcca caccaccatg tacttcatgt 780

gtgagtttga caaggagaac atgtgagcct caggctgggg ctgcccattg ggggccccac 840

atgtccctgc agggttggca gggacagagc ccagaccatg gtgccagcca gggagctgtc 900

cctctgtgaa gggtggaggc tcactgagta gagggctgtt gtctaaactg agaaaatggc 960

ctatgcttaa gaggaaaatg aaagtgttcc tggggtgctg tctctgaaga agcagagttt 1020

cattacctgt attgtagccc caatgtcatt atgtaattat tacccagaat tgctcttcca 1080

taaagcttgt gcctttgtcc aagctataca ataaaatctt taagtagtgc agta 1134

<210> 25
<211> 1259
<212> DNA
<213> Homo sapiens

<400> 25
gccatgaggc gcctgggggc agtgtcctcg cgggccagcg acgggcagga cgccccgttc 60

gcctagcgcg tgctcaggag ttggtgtcct gcctgcgctc aggatgaggg ggaatctggc 120

cctggtgggc gttctaatca gcctggcctt cctgtcactg ctgccatctg gacatcctca 180

gccggctggc gatgacgcct gctctgtgca gatcctcgtc cctggcctca aaggggatgc 240

gggagagaag ggagacaaag gcgcccccgg acggcctgga agagtcggcc ccacgggaga 300

aaaaggagac atgggggaca aaggacagaa aggcagtgtg ggtcgtcatg gaaaaattgg 360

tcccattggc tctaaaggtg agaaaggaga ttccggtgac ataggacccc ctggtcctaa 420

tggagaacca ggcctcccat gtgagtgcag ccagctgcgc aaggccatcg gggagatgga 480

caaccaggtc tctcagctga ccagcgagct caagttcatc aagaatgctg tcgccggtgt 540

gcgcgagacg gagagcaaga tctacctgct ggtgaaggag gagaagcgct acgcggacgc 600

ccagctgtcc tgccagggcc gcgggggcac gctgagcatg cccaaggacg aggctgccaa 660

tggcctgatg gccgcatacc tggcgcaagc cggcctggcc cgtgtcttca tcggcatcaa 720

cgacctggag aaggagggcg ccttcgtgta ctctgaccac tcccccatgc ggaccttcaa 780

caagtggcgc agcggtgagc ccaacaatgc ctacgacgag gaggactgcg tggagatggt 840

ggcctcgggc ggctggaacg acgtggcctg ccacaccacc atgtacttca tgtgtgagtt 900

tgacaaggag aacatgtgag cctcaggctg gggctgccca ttgggggccc cacatgtccc 960

tgcagggttg gcagggacag agcccagacc atggtgccag ccagggagct gtccctctgt 1020

gaagggtgga ggctcactga gtagagggct gttgtctaaa ctgagaaaat ggcctatgct 1080

taagaggaaa atgaaagtgt tcctggggtg ctgtctctga agaagcagag tttcattacc 1140

tgtattgtag ccccaatgtc attatgtaat tattacccag aattgctctt ccataaagct 1200

tgtgcctttg tccaagctat acaataaaat ctttaagtag tgcagtagtt aagtccaaa 1259

<210> 26
<211> 1381
<212> DNA
<213> Homo sapiens

<400> 26
cttctccctg cgcctgggac tgcggccgcc tctctaaata gcagccatga ggcgcctggg 60

ggcagtgtcc tcgcgggcca gcgacgggca ggacgccccg ttcgcctagc gcgtgctcag 120

gagttggtgt cctgcctgcg ctcaggatga gggggaatct ggccctggtg ggcgttctaa 180

tcagcctggc cttcctgtca ctgctgccat ctggacatcc tcagccggct ggcgatgacg 240

cctgctctgt gcagatcctc gtccctggcc tcaaagggga tgcgggagag aagggagaca 300

aaggcgcccc cggacggcct ggaagagtcg gccccacggg agaaaaagca gtcacagcca 360

gtcgtgacag cttctgacat ctccaaaagg aagtgcacct cgtcctttgt agaaatggga 420

tcacagggag acatggggga caaaggacag aaaggcagtg tgggtcgtca tggaaaaatt 480

ggtcccattg gctctaaagg tgagaaagga gattccggtg acataggacc ccctggtcct 540

aatggagaac caggcctccc atgtgagtgc agccagctgc gcaaggccat cggggagatg 600

gacaaccagg tctctcagct gaccagcgag ctcaagttca tcaagaatgc tgtcgccggt 660

gtgcgcgaga cggagagcaa gatctacctg ctggtgaagg aggagaagcg ctacgcggac 720

gcccagctgt cctgccaggg ccgcgggggc acgctgagca tgcccaagga cgaggctgcc 780

aatggcctga tggccgcata cctggcgcaa gccggcctgg cccgtgtctt catcggcatc 840

aacgacctgg agaaggaggg cgccttcgtg tactctgacc actcccccat gcggaccttc 900

aacaagtggc gcagcggtga gcccaacaat gcctacgacg aggaggactg cgtggagatg 960

gtggcctcgg gcggctggaa cgacgtggcc tgccacacca ccatgtactt catgtgtgag 1020

tttgacaagg agaacatgtg agcctcaggc tggggctgcc cattgggggc cccacatgtc 1080

cctgcagggt tggcagggac agagcccaga ccatggtgcc agccagggag ctgtccctct 1140

gtgaagggtg gaggctcact gagtagaggg ctgttgtcta aactgagaaa atggcctatg 1200

cttaagagga aaatgaaagt gttcctgggg tgctgtctct gaagaagcag agtttcatta 1260

cctgtattgt agccccaatg tcattatgta attattaccc agaattgctc ttccataaag 1320

cttgtgcctt tgtccaagct atacaataaa atctttaagt agtgcagtag ttaagtccaa 1380

a 1381

<210> 27
<211> 1311
<212> DNA
<213> Homo sapiens

<400> 27
ggacggtgga cgcagcgcag acaggaagct ccccgagata acgctgcggc cgggcggcct 60

gatttgctgg gctgtctgat ggcccgggcc gaggcttctc cctgcgcctg ggactgcggc 120

cgcctctcta aatagcagcc atgaggcgcc tgggggcagt gtcctcgcgg gccagcgacg 180

ggcaggacgc cccgttcgcc tagcgcgtgc tcaggagttg gtgtcctgcc tgcgctcagg 240

atgaggggga atctggccct ggtgggcgtt ctaatcagcc tggccttcct gtcactgctg 300

ccatctggac atcctcagcc ggctggcgat gacgcctgct ctgtgcagat cctcgtccct 360

ggcctcaaag gggatgcggg agagaaggga gacaaaggcg cccccggacg gcctggaaga 420

gtcggcccca cgggagaaaa aggtgagaaa ggagattccg gtgacatagg accccctggt 480

cctaatggag aaccaggcct cccatgtgag tgcagccagc tgcgcaaggc catcggggag 540

atggacaacc aggtctctca gctgaccagc gagctcaagt tcatcaagaa tgctgtcgcc 600

ggtgtgcgcg agacggagag caagatctac ctgctggtga aggaggagaa gcgctacgcg 660

gacgcccagc tgtcctgcca gggccgcggg ggcacgctga gcatgcccaa ggacgaggct 720

gccaatggcc tgatggccgc atacctggcg caagccggcc tggcccgtgt cttcatcggc 780

atcaacgacc tggagaagga gggcgccttc gtgtactctg accactcccc catgcggacc 840

ttcaacaagt ggcgcagcgg tgagcccaac aatgcctacg acgaggagga ctgcgtggag 900

atggtggcct cgggcggctg gaacgacgtg gcctgccaca ccaccatgta cttcatgtgt 960

gagtttgaca aggagaacat gtgagcctca ggctggggct gcccattggg ggccccacat 1020

gtccctgcag ggttggcagg gacagagccc agaccatggt gccagccagg gagctgtccc 1080

tctgtgaagg gtggaggctc actgagtaga gggctgttgt ctaaactgag aaaatggcct 1140

atgcttaaga ggaaaatgaa agtgttcctg gggtgctgtc tctgaagaag cagagtttca 1200

ttacctgtat tgtagcccca atgtcattat gtaattatta cccagaattg ctcttccata 1260

aagcttgtgc ctttgtccaa gctatacaat aaaatcttta agtagtgcag t 1311

<210> 28
<211> 1383

<212>  DNA
<213>  Homo sapiens

<400>  28
ggacggtgga cgcagcgcag acaggaagct ccccgagata acgctgcggc cgggcggcct      60

gatttgctgg gctgtctgat ggcccgggcc gaggcttctc cctgcgcctg ggactgcggc     120

cgcctctcta aatagcagcc atgaggcgcc tgggggcagt gtcctcgcgg gccagcgacg     180

ggcaggacgc cccgttcgcc tagcgcgtgc tcaggagttg gtgtcctgcc tgcgctcagg     240

atgaggggga atctggccct ggtgggcgtt ctaatcagcc tggccttcct gtcactgctg     300

ccatctggac atcctcagcc ggctggcgat gacgcctgct ctgtgcagat cctcgtccct     360

ggcctcaaag gggatgcggg agagaaggga gacaaaggcg cccccggacg gcctggaaga     420

gtcggcccca cgggagaaaa aggagacatg ggggacaaag gacagaaagg cagtgtgggt     480

cgtcatggaa aaattggtcc cattggctct aaaggtgaga aaggagattc cggtgacata     540

ggaccccctg gtcctaatgg agaaccaggc ctcccatgtg agtgcagcca gctgcgcaag     600

gccatcgggg agatggacaa ccaggtctct cagctgacca gcgagctcaa gttcatcaag     660

aatgctgtcg ccggtgtgcg cgagacggag agcaagatct acctgctggt gaaggaggag     720

aagcgctacg cggacgccca gctgtcctgc cagggccgcg ggggcacgct gagcatgccc     780

aaggacgagg ctgccaatgg cctgatggcc gcatacctgg cgcaagccgg cctggcccgt     840

gtcttcatcg gcatcaacga cctggagaag gagggcgcct tcgtgtactc tgaccactcc     900

cccatgcgga ccttcaacaa gtggcgcagc ggtgagccca acaatgccta cgacgaggag     960

gactgcgtgg agatggtggc ctcgggcggc tggaacgacg tggcctgcca caccaccatg    1020

tacttcatgt gtgagtttga caaggagaac atgtgagcct caggctgggg ctgcccattg    1080

ggggccccac atgtccctgc agggttggca gggacagagc ccagaccatg gtgccagcca    1140

gggagctgtc cctctgtgaa gggtggaggc tcactgagta gagggctgtt gtctaaactg    1200

agaaaatggc ctatgcttaa gaggaaaatg aaagtgttcc tggggtgctg tctctgaaga    1260

agcagagttt cattacctgt attgtagccc caatgtcatt atgtaattat tacccagaat    1320

tgctcttcca taaagcttgt gcctttgtcc aagctataca ataaaatctt taagtagtgc    1380

agt                                                                  1383


<210>  29
<211>  1462
<212>  DNA
<213>  Homo sapiens

<400>  29
ggacggtgga cgcagcgcag acaggaagct ccccgagata acgctgcggc cgggcggcct      60

gatttgctgg gctgtctgat ggcccgggcc gaggcttctc cctgcgcctg ggactgcggc     120

cgcctctcta aatagcagcc atgaggcgcc tgggggcagt gtcctcgcgg gccagcgacg    180

ggcaggacgc cccgttcgcc tagcgcgtgc tcaggagttg gtgtcctgcc tgcgctcagg    240

atgaggggga atctggccct ggtgggcgtt ctaatcagcc tggccttcct gtcactgctg    300

ccatctggac atcctcagcc ggctggcgat gacgcctgct ctgtgcagat cctcgtccct    360

ggcctcaaag gggatgcggg agagaaggga gacaaaggcg cccccggacg gcctggaaga    420

gtcggcccca cgggagaaaa agcagtcaca gccagtcgtg acagcttctg acatctccaa    480

aaggaagtgc acctcgtcct ttgtagaaat gggatcacag ggagacatgg gggacaaagg    540

acagaaaggc agtgtgggtc gtcatggaaa aattggtccc attggctcta aaggtgagaa    600

aggagattcc ggtgacatag gaccccctgg tcctaatgga gaaccaggcc tcccatgtga    660

gtgcagccag ctgcgcaagg ccatcgggga gatggacaac caggtctctc agctgaccag    720

cgagctcaag ttcatcaaga atgctgtcgc cggtgtgcgc gagacggaga gcaagatcta    780

cctgctggtg aaggaggaga agcgctacgc ggacgcccag ctgtcctgcc agggccgcgg    840

gggcacgctg agcatgccca aggacgaggc tgccaatggc ctgatggccg catacctggc    900

gcaagccggc ctggcccgtg tcttcatcgg catcaacgac ctggagaagg agggcgcctt    960

cgtgtactct gaccactccc ccatgcggac cttcaacaag tggcgcagcg gtgagcccaa   1020

caatgcctac gacgaggagg actgcgtgga gatggtggcc tcgggcggct ggaacgacgt   1080

ggcctgccac accaccatgt acttcatgtg tgagtttgac aaggagaaca tgtgagcctc   1140

aggctggggc tgcccattgg gggccccaca tgtccctgca gggttggcag ggacagagcc   1200

cagaccatgg tgccagccag ggagctgtcc ctctgtgaag ggtggaggct cactgagtag   1260

agggctgttg tctaaactga gaaaatggcc tatgcttaag aggaaaatga aagtgttcct   1320

ggggtgctgt ctctgaagaa gcagagtttc attacctgta ttgtagcccc aatgtcatta   1380

tgtaattatt acccagaatt gctcttccat aaagcttgtg cctttgtcca agctatacaa   1440

taaaatcttt aagtagtgca gt                                            1462

<210>  30
<211>  1240
<212>  DNA
<213>  Homo sapiens

<400>  30
ggacggtgga cgcagcgcag acaggaagct ccccgagata acgctgcggc cgggcggcct     60

gatttgctgg gctgtctgat ggcccgggcc gaggcttctc cctgcgcctg ggactgcggc    120

cgcctctcta aatagcagcc atgaggcgcc tgggggcagt gtcctcgcgg gccagcgacg    180

ggcaggacgc cccgttcgcc tagcgcgtgc tcaggagttg gtgtcctgcc tgcgctcagg    240

atgaggggga atctggccct ggtgggcgtt ctaatcagcc tggccttcct gtcactgctg    300

ccatctggac atcctcagcc ggctggcgat gacgcctgct ctgtgcagat cctcgtccct 360

ggcctcaaag gtgagaaagg agattccggt gacataggac cccctggtcc taatggagaa 420

ccaggcctcc catgtgagtg cagccagctg cgcaaggcca tcggggagat ggacaaccag 480

gtctctcagc tgaccagcga gctcaagttc atcaagaatg ctgtcgccgg tgtgcgcgag 540

acggagagca agatctacct gctggtgaag gaggagaagc gctacgcgga cgcccagctg 600

tcctgccagg gccgcggggg cacgctgagc atgcccaagg acgaggctgc caatggcctg 660

atggccgcat acctggcgca agccggcctg gcccgtgtct tcatcggcat caacgacctg 720

gagaaggagg gcgccttcgt gtactctgac cactccccca tgcggacctt caacaagtgg 780

cgcagcggtg agcccaacaa tgcctacgac gaggaggact gcgtggagat ggtggcctcg 840

ggcggctgga acgacgtggc ctgccacacc accatgtact tcatgtgtga gtttgacaag 900

gagaacatgt gagcctcagg ctggggctgc ccattggggg ccccacatgt ccctgcaggg 960

ttggcaggga cagagcccag accatggtgc cagccaggga gctgtccctc tgtgaagggt 1020

ggaggctcac tgagtagagg gctgttgtct aaactgagaa aatggcctat gcttaagagg 1080

aaaatgaaag tgttcctggg gtgctgtctc tgaagaagca gagtttcatt acctgtattg 1140

tagccccaat gtcattatgt aattattacc cagaattgct cttccataaa gcttgtgcct 1200

ttgtccaagc tatacaataa aatctttaag tagtgcagta 1240

<210> 31
<211> 1312
<212> DNA
<213> Homo sapiens

<400> 31
ggacggtgga cgcagcgcag acaggaagct ccccgagata acgctgcggc cgggcggcct 60

gatttgctgg gctgtctgat ggcccgggcc gaggcttctc cctgcgcctg ggactgcggc 120

cgcctctcta aatagcagcc atgaggcgcc tgggggcagt gtcctcgcgg gccagcgacg 180

ggcaggacgc cccgttcgcc tagcgcgtgc tcaggagttg gtgtcctgcc tgcgctcagg 240

atgaggggga atctggccct ggtgggcgtt ctaatcagcc tggccttcct gtcactgctg 300

ccatctggac atcctcagcc ggctggcgat gacgcctgct ctgtgcagat cctcgtccct 360

ggcctcaaag gagacatggg ggacaaagga cagaaaggca gtgtgggtcg tcatggaaaa 420

attggtccca ttggctctaa aggtgagaaa ggagattccg gtgacatagg accccctggt 480

cctaatggag aaccaggcct cccatgtgag tgcagccagc tgcgcaaggc catcggggag 540

atggacaacc aggtctctca gctgaccagc gagctcaagt tcatcaagaa tgctgtcgcc 600

ggtgtgcgcg agacggagag caagatctac ctgctggtga aggaggagaa gcgctacgcg 660

gacgcccagc tgtcctgcca gggccgcggg ggcacgctga gcatgcccaa ggacgaggct 720

gccaatggcc tgatggccgc atacctggcg caagccggcc tggcccgtgt cttcatcggc 780

atcaacgacc tggagaagga gggcgccttc gtgtactctg accactcccc catgcggacc 840

ttcaacaagt ggcgcagcgg tgagcccaac aatgcctacg acgaggagga ctgcgtggag 900

atggtggcct cgggcggctg gaacgacgtg gcctgccaca ccaccatgta cttcatgtgt 960

gagtttgaca aggagaacat gtgagcctca ggctggggct gcccattggg ggccccacat 1020

gtccctgcag ggttggcagg gacagagccc agaccatggt gccagccagg gagctgtccc 1080

tctgtgaagg gtggaggctc actgagtaga gggctgttgt ctaaactgag aaaatggcct 1140

atgcttaaga ggaaaatgaa agtgttcctg gggtgctgtc tctgaagaag cagagtttca 1200

ttacctgtat tgtagcccca atgtcattat gtaattatta cccagaattg ctcttccata 1260

aagcttgtgc ctttgtccaa gctatacaat aaaatcttta agtagtgcag ta 1312

<210> 32
<211> 4834
<212> DNA
<213> Homo sapiens

<400> 32
gcctagggag gaggagcgag aatcagcctg gttcagtgac tgagacaaac tggaggtgaa 60

aggagctggt actgtccact gtgctgtcgg tgctgaacct gagacgcgag cggaccagtt 120

gctccagcac ctgaaggcaa cgccctcttg caccctctgt gccctgtggg acccgcttca 180

ccaacaggac ccatatcaac ttgacaaagg agtgtggtat cggacgtggg agagagtcct 240

ctgtttgcca cctgggcgct cattcaggcg tgactttgga gatttctata gttttagacc 300

aaactatttt tttttcccca gctaagacga tcttttgaga gttttttttt tttattgtga 360

tttatatttc cacagcgttt aggaatcttt ctgggggact tttgtgactg ttaaaataag 420

gtgaaaagca ataaggatgt ttaagtgctg gtcagttgtc ttggttctcg gattcatttt 480

tctggagtcg gaaggaaggc caaccaaaga aggaggatat ggccttaaat cctatcagcc 540

tctaatgaga ttgcgacata agcaggaaaa aaatcaagaa agttcaagag tcaaaggatt 600

tatgattcag gatggccctt ttggatcttg tgaaaataag tactgtggtt tgggaagaca 660

ctgtgttacc agcagagaga cagggcaagc agaatgtgcc tgtatggacc tttgcaaacg 720

tcactacaaa cctgtgtgtg gatctgacgg agaattctat gaaaaccact gtgaagtgca 780

cagagctgct tgcctgaaaa aacaaaagat taccattgtt cacaatgaag actgcttctt 840

taaaggagat aagtgcaaga ctactgaata cagcaagatg aaaaatatgc tattagattt 900

acaaaatcaa aaatatatta tgcaagaaaa tgaaaatcct aatggcgacg acatatctcg 960

gaagaagcta ttggtggatc aaatgtttaa atattttgat gcagacagta atggacttgt 1020

agatattaat gaactaactc aggtgataaa acaggaagaa cttggcaagg atctctttga 1080

ttgtactttg tatgttctat tgaaatatga tgattttaat gctgacaagc acctggctct 1140

tgaagaattt tatagagcat tccaagtgat ccagttgagt ctgccagaag atcagaaact 1200

aagcatcact gcagcaactg tgggacaaag tgctgttctg agctgtgcca ttcaaggaac 1260

cctgagacct cccattatct ggaaaaggaa caatattatt ctaaataatt tagatttgga 1320

agacatcaat gactttggag atgatgggtc cttgtatatt actaaggtta ccacaactca 1380

cgttggcaat tacacctgct atgcagatgg ctatgaacaa gtctatcaga ctcacatctt 1440

ccaagtgaat gttcctccag tcatccgggt gtatccagag agtcaggcta gagagcctgg 1500

ggtaactgcc agtcttaggt gccatgcaga gggcatacca aagcctcagc ttggctggtt 1560

gaagaatgga attgatatta caccaaagct ttccaaacaa ctcacgcttc aagcaaatgg 1620

cagtgaggtt cacataagca atgtgcgcta tgaagatact ggagcataca cttgtatcgc 1680

aaagaatgaa gcaggagtgg atgaagacat ctcttctctt tttgtggaag actctgctag 1740

aaagacccta gctaacatat tatggagaga agaaggtctg ggaattggga acatgttcta 1800

tgttttttat gaagatggaa tcaaagtgat acaacccata gaatgtgaat ttcagaggca 1860

cattaagcct agtgaaaagc tccttggatt tcaggcaaag gatgaagtct gtcccaaagc 1920

tgagggagat gaagttcaga ggtgtgtgtg ggcatcagct gttaatgtca aagacaagtt 1980

catttatgtt gcacagccaa ctttggacag agtccttatt gttgatgtgc agtcccaaaa 2040

agttgttcag gcagtgagca cagaccctgt cccagttaaa ttacactatg acaaatcaca 2100

tgatcaggtc tgggtgctaa gctggggtac cttggagaag acatcaccaa cactacaggt 2160

aattaccctg gccagtggga atgtgcctca ccacacgatc cacacccaac cagtgggaaa 2220

gcaatttgac agagtggatg attttttcat tcccaccaca acactcatta tcacccatat 2280

gaggtttgga tttattcttc ataaagatga agctgcacta caaaaaattg atcttgaaac 2340

catgtcatac atcaagacaa ttaacttgaa ggactataag tgcgttcctc agtcattggc 2400

atatacacac ttgggaggct actacttcat tggctgcaaa cctgacagca ccggagcagt 2460

ttccccacag gtcatggtgg acggtgtaac tgactcagtc attgggttca atagtgatgt 2520

gacgggcact ccatatgtct ctccagatgg ccactacctt gtcagcatta atgatgtgaa 2580

aggtcttgta agggttcagt acattaccat cagaggagaa atacaggagg cttttgatat 2640

ttacacaaat ctgcacatat ctgatctggc atttcaacca tcctttactg aagcccacca 2700

atataacatc tacggtagtt caagcacaca aactgatgtg ctctttgtgg agctctcttc 2760

tgggaaggtc aagatgataa agagtctcaa ggaaccactc aaggcagaag aatggccttg 2820

gaaccggaaa aacaggcaaa tccaggacag tggcttgttt ggtcaatacc tgatgacacc 2880

ttccaaggac tctctcttca tcctagatgg acgactcaat aaattaaact gtgagatcac 2940

tgaagttgaa aaaggaaata cagtcatttg ggttggagat gcctaaaaac cctacgatac 3000

aattattgaa tgaagcgttt tacaatacat tgcacttaat ccattgttta aatttacaac 3060

ttaactttcc aagtttatat cctagtcaaa caaaatttac ttggttggtc caaataaaat 3120

aaattgtttt tgactaagtg actaagacat acacaattaa ttatattggt taattagaaa 3180

tactcagcac aatatttaat gagaaatcac atcagtcaca agaatcaaat atgtaaatta 3240

tgctttaaac aataaagtag gatttaaatc atttaaaaaa ctaaactgca gggaatttca 3300

gatgacataa agcattattt cattttgagg gtagtttatt ttttttctct gtccttttgg 3360

tatgattaaa caagaaataa aaggtgttgt ggaaacttaa aattcttaat tcaaaatatt 3420

cggtcctgtc cactgtttat aatattttgt gccttgaagg gaatgtcaca ggaggaaaag 3480

gaagactaaa gcctgcatac taaatgctct cataactata aatctcttgt tgttaaaaaa 3540

catctcttgc accaaatcct gtcttcagtc ttttgtttaa ccctcaatga actaccaatt 3600

gaaggcataa gttggtctta ggttgtatac aggtgtcaaa ctccacttga catttactgt 3660

ataatctgca gcatgctata gaaagtgggg aggaagggag gaaaaataat tacagtcaaa 3720

atatttgcaa tctatctact cattttgctt tcaatcacta gagtgcatta tgtttttaat 3780

gcaagtttgt ctgggatgtc aactgcctac agatgtttat tactagcaga atagattatt 3840

tattaataga ttgtttcttt tcatatagtc caatgtttca tcatcaggaa ctttcggttt 3900

taaaaaagaa acattacttt tgtctgctaa agtaaaccat atatagggaa attaacacct 3960

aggaaaagaa aactggcaag agaattgatg aggtattagt ggcttagaag aattagtaat 4020

ataagttggg aataaagggg tagcccgaca gtggtaagta aaaatgtagt actccattgt 4080

ttttgctatt taaatataat gtatcaaaaa cctttattct aaaatttcca caaatgattc 4140

cttttatcca tcttcttgta atcctaaaag catcttctca ctatgtgctg tagtttgaaa 4200

cctaacgaat cctcctactt gagacactgt agaaaagtaa aaattgttgc ttctgttttg 4260

ttttaagagg aaatgagaag gccagcagaa gtagattaaa actagagagc tggtttatta 4320

aaataagcag ataattatcc aggcttaaag taatagctta catgttttga aaatccttgt 4380

acttattttt gtttttattg ttgcaaaaaa atgttaatgg gctgttgaaa tgacctccat 4440

ttacttaagg taatccaata aggtcttttt taaatataca aattttactc aagtttaaat 4500

tgtataaaac tgcttagaaa cctgaaattt tatagtgtgt aatatcttat gtgtatatag 4560

catcctgagc tgtgtgatac agtagatttc ttcttagcag aaacatgcga ggtagattta 4620

ataaacaaca aaaatcagct tatgtttcta cttattgaat ctgctttact ttttagcaat 4680

tttcttcctt gtgaacatga accatataaa gtgtctgtga cttaatgatt tcatgtaagt 4740

ctgtcgcata catccttctt ttgtagctta caccatgtgg atgctctgtc tgtagacact 4800

gcttgataaa aggagaataa acctcttaca tttt                                4834

<210>  33
<211>  4828
<212>  DNA
<213>  Homo sapiens

<400>  33
gcctagggag gaggagcgag aatcagcctg gttcagtgac tgagacaaac tggaggtgaa      60

aggagctggt actgtccact gtgctgtcgg tgctgaacct gagacgcgag cggaccagtt     120

gctccagcac ctgaaggcaa cgccctcttg caccctctgt gccctgtggg acccgcttca     180

ccaacaggac ccatatcaac ttgacaaagg agtgtggtat cggacgtggg agagagtcct     240

ctgtttgcca cctgggcgct cattcaggcg tgactttgga gatttctata gttttagacc     300

aaactatttt tttttcccca gctaagacga tcttttgaga gttttttttt tttattgtga     360

tttatatttc cacagcgttt aggaatcttt ctgggggact tttgtgactg ttaaaataag     420

gtgaaaagca ataaggatgt ttaagtgctg gtcagttgtc ttggttctcg gattcatttt     480

tctggagtcg gaaggaaggc caaccaaaga aggaggatat ggccttaaat cctatcagcc     540

tctaatgaga ttgcgacata agcaggaaaa aaatcaagaa agttcaagag tcaaaggatt     600

tatgattcag gatggccctt ttggatcttg tgaaaataag tactgtggtt tgggaagaca     660

ctgtgttacc agcagagaga cagggcaagc agaatgtgcc tgtatggacc tttgcaaacg     720

tcactacaaa cctgtgtgtg gatctgacgg agaattctat gaaaaccact gtgaagtgca     780

cagagctgct tgcctgaaaa aacaaaagat taccattgtt cacaatgaag actgcttctt     840

taaaggagat aagtgcaaga ctactgaata cagcaagatg aaaaatatgc tattagattt     900

acaaaatcaa aaatatatta tgcaagaaaa tgaaaatcct aatggcgacg acatatctcg     960

gaagaagcta ttggtggatc aaatgtttaa atattttgat gcagacagta atggacttgt    1020

agatattaat gaactaactc aggtgataaa acaggaagaa cttggcaagg atctctttga    1080

ttgtactttg tatgttctat tgaaatatga tgattttaat gctgacaagc acctggctct    1140

tgaagaattt tatagagcat tccaagtgat ccagttgagt ctgccagaag atcagaaact    1200

aagcatcact gcagcaactg tgggacaaag tgctgttctg agctgtgcca ttcaaggaac    1260

cctgagacct cccattatct ggaaaaggaa caatattatt ctaaataatt tagatttgga    1320

agacatcaat gactttggag atgatgggtc cttgtatatt actaaggtta ccacaactca    1380

cgttggcaat tacacctgct atgcagatgg ctatgaacaa gtctatcaga ctcacatctt    1440

ccaagtgaat gttcctccag tcatccgggt gtatccagag agtcaggcta gagagcctgg    1500

ggtaactgcc agtcttaggt gccatgcaga gggcatacca aagcctcagc ttggctggtt    1560

gaagaatgga attgatatta caccaaagct ttccaaacaa ctcacgcttc aagcaaatgg    1620

cagtgaggtt cacataagca atgtgcgcta tgaagatact ggagcataca cttgtatcgc 1680

aaagaatgaa gcaggagtgg atgaagacat ctcttctctt tttgtggaag actctgctag 1740

aaagacccta gctaacatat tatggagaga agaaggtctg ggaattggga acatgttcta 1800

tgttttttat gaagatggaa tcaaagtgat acaacccata gaatgtgaat ttcagaggca 1860

cattaagcct agtgaaaagc tccttggatt tcaggatgaa gtctgtccca aagctgaggg 1920

agatgaagtt cagaggtgtg tgtgggcatc agctgttaat gtcaaagaca agttcattta 1980

tgttgcacag ccaactttgg acagagtcct tattgttgat gtgcagtccc aaaaagttgt 2040

tcaggcagtg agcacagacc ctgtcccagt taaattacac tatgacaaat cacatgatca 2100

ggtctgggtg ctaagctggg gtaccttgga gaagacatca ccaacactac aggtaattac 2160

cctggccagt gggaatgtgc ctcaccacac gatccacacc caaccagtgg gaaagcaatt 2220

tgacagagtg gatgattttt tcattcccac cacaacactc attatcaccc atatgaggtt 2280

tggatttatt cttcataaag atgaagctgc actacaaaaa attgatcttg aaaccatgtc 2340

atacatcaag acaattaact tgaaggacta taagtgcgtt cctcagtcat tggcatatac 2400

acacttggga ggctactact tcattggctg caaacctgac agcaccggag cagtttcccc 2460

acaggtcatg gtggacggtg taactgactc agtcattggg ttcaatagtg atgtgacggg 2520

cactccatat gtctctccag atggccacta ccttgtcagc attaatgatg tgaaaggtct 2580

tgtaagggtt cagtacatta ccatcagagg agaaatacag gaggcttttg atatttacac 2640

aaatctgcac atatctgatc tggcatttca accatccttt actgaagccc accaatataa 2700

catctacggt agttcaagca cacaaactga tgtgctcttt gtggagctct cttctgggaa 2760

ggtcaagatg ataaagagtc tcaaggaacc actcaaggca gaagaatggc cttggaaccg 2820

gaaaaacagg caaatccagg acagtggctt gtttggtcaa tacctgatga caccttccaa 2880

ggactctctc ttcatcctag atggacgact caataaatta aactgtgaga tcactgaagt 2940

tgaaaaagga aatacagtca tttgggttgg agatgcctaa aaaccctacg atacaattat 3000

tgaatgaagc gttttacaat acattgcact taatccattg tttaaattta caacttaact 3060

ttccaagttt atatcctagt caaacaaaat ttacttggtt ggtccaaata aaataaattg 3120

tttttgacta agtgactaag acatacacaa ttaattatat tggttaatta gaaatactca 3180

gcacaatatt taatgagaaa tcacatcagt cacaagaatc aaatatgtaa attatgcttt 3240

aaacaataaa gtaggattta aatcatttaa aaaactaaac tgcagggaat ttcagatgac 3300

ataaagcatt atttcatttt gagggtagtt tatttttttt ctctgtcctt ttggtatgat 3360

taaacaagaa ataaaaggtg ttgtggaaac ttaaaattct taattcaaaa tattcggtcc 3420

tgtccactgt ttataatatt ttgtgccttg aagggaatgt cacaggagga aaaggaagac 3480

taaagcctgc atactaaatg ctctcataac tataaatctc ttgttgttaa aaaacatctc 3540

ttgcaccaaa tcctgtcttc agtcttttgt ttaaccctca atgaactacc aattgaaggc 3600

ataagttggt cttaggttgt atacaggtgt caaactccac ttgacattta ctgtataatc 3660

tgcagcatgc tatagaaagt ggggaggaag ggaggaaaaa taattacagt caaaatattt 3720

gcaatctatc tactcatttt gctttcaatc actagagtgc attatgtttt taatgcaagt 3780

ttgtctggga tgtcaactgc ctacagatgt ttattactag cagaatagat tatttattaa 3840

tagattgttt cttttcatat agtccaatgt ttcatcatca ggaactttcg gttttaaaaa 3900

agaaacatta cttttgtctg ctaaagtaaa ccatatatag ggaaattaac acctaggaaa 3960

agaaaactgg caagagaatt gatgaggtat tagtggctta gaagaattag taatataagt 4020

tgggaataaa ggggtagccc gacagtggta agtaaaaatg tagtactcca ttgtttttgc 4080

tatttaaata taatgtatca aaaaccttta ttctaaaatt tccacaaatg attcctttta 4140

tccatcttct tgtaatccta aaagcatctt ctcactatgt gctgtagttt gaaacctaac 4200

gaatcctcct acttgagaca ctgtagaaaa gtaaaaattg ttgcttctgt tttgttttaa 4260

gaggaaatga gaaggccagc agaagtagat taaaactaga gagctggttt attaaaataa 4320

gcagataatt atccaggctt aaagtaatag cttacatgtt ttgaaaatcc ttgtacttat 4380

ttttgttttt attgttgcaa aaaaatgtta atgggctgtt gaaatgacct ccatttactt 4440

aaggtaatcc aataaggtct tttttaaata tacaaatttt actcaagttt aaattgtata 4500

aaactgctta gaaacctgaa attttatagt gtgtaatatc ttatgtgtat atagcatcct 4560

gagctgtgtg atacagtaga tttcttctta gcagaaacat gcgaggtaga tttaataaac 4620

aacaaaaatc agcttatgtt tctacttatt gaatctgctt tactttttag caattttctt 4680

ccttgtgaac atgaaccata taaagtgtct gtgacttaat gatttcatgt aagtctgtcg 4740

catacatcct tcttttgtag cttacaccat gtggatgctc tgtctgtaga cactgcttga 4800

taaaaggaga ataaacctct tacatttt 4828

<210> 34
<211> 2066
<212> DNA
<213> Homo sapiens

<400> 34
atgtctagac tgggagccct gggtggtgcc cgtgccgggc tgggactgtt gctgggtacc 60

gccgccggcc ttggattcct gtgcctcctt tacagccagc gatggaaacg gacccagcgt 120

catggccgca gccagagcct gcccaactcc ctggactata cgcagacttc agatcccgga 180

cgccacgtga tgctcctgcg ggctgtccca ggtggggctg gagatgcctc agtgctgccc 240

agccttccac gggaaggaca ggagaaggtg ctggaccgcc tggactttgt gctgaccagc 300

cttgtggcgc tgcggcggga ggtggaggag ctgagaagca gcctgcgagg gcttgcgggg 360

gagattgttg gggaggtccg atgccacatg gaagagaacc agagagtggc tcggcggcga 420

aggtttccgt ttgtccggga gaggagtgac tccactggct ccagctctgt ctacttcacg 480

gcctcctcgg gagccacgtt cacagatgct gagagtgaag ggggttacac aacagccaat 540

gcggagtctg acaatgagcg ggactctgac aaagaaagtg aggacgggga agatgaagtg 600

agctgtgaga ctgtgaagat ggggagaaag gattctcttg acttggagga agaggcagct 660

tcaggtgcct ccagtgccct ggaggctgga ggttcctcag gcttggagga tgtgctgccc 720

ctcctgcagc aggccgacga gctgcacagg ggtgatgagc aaggcaagcg ggagggcttc 780

cagctgctgc tcaacaacaa gctggtgtat ggaagccggc aggactttct ctggcgcctg 840

gcccgagcct acagtgacat gtgtgagctc actgaggagg tgagcgagaa gaagtcatat 900

gccctagatg gaaaagaaga agcagaggct gctctggaga agggggatga gagtgctgac 960

tgtcacctgt ggtatgcggt gctttgtggt cagctggctg agcatgagag catccagagg 1020

cgcatccaga gtggctttag cttcaaggag catgtggaca aagccattgc tctccagcca 1080

gaaaacccca tggctcactt tcttcttggc aggtggtgct atcaggtctc tcacctgagc 1140

tggctagaaa aaaaaactgc tacagccttg cttgaaagcc ctctcagtgc cactgtggaa 1200

gatgccctcc agagcttcct aaaggctgaa gaactacagc caggattttc caaagcagga 1260

agggtatata tttccaagtg ctacagagaa ctagggaaaa actctgaagc tagatggtgg 1320

atgaagttgg ccctggagct gccagatgtc acgaaggagg atttggctat ccagaaggac 1380

ctggaagaac tggaagtcat tttacgagac taaccacgtt tcactggcct tcatgacttg 1440

atgccactat ttaaggtggg ggggcgggga ggcttttttc cttagacctt gctgagatca 1500

ggaaaccaca caaatctgtc tcctgggtct gactgctacc cactaccact ccccattagt 1560

taatttattc taacctctaa cctaatctag aattggggca gtactcatgg cttccgtttc 1620

tgttgttctc tcccttgagt aatctcttaa aaaaatcaag attcacacct gccccaggat 1680

tacacatggg tagagcctgc aagacctgag accttccaat tgctggtgag gtggatgaac 1740

ttcaaagcta taggaacaaa gcacataact tgtcacttta atctttttca ctgactaata 1800

ggactcagta catatagtct taagatcata ccttacctac caaggtaaaa agagggatca 1860

gagtggccca cagacattgc tttcttatca cctatcatgt gaattctacc tgtattcctg 1920

ggctggacca cttgataact tccagtgtcc tggcagcttt tggaatgaca gcagtggtat 1980

ggggtttatg atgctataaa acaatgtctg aaaagttgcc tagaatatat tttgttacaa 2040

acttgaaata aaccaaattt gatgtt 2066

<210> 35

<211>  2251
<212>  DNA
<213>  Homo sapiens

<400>  35
gacgtcacgg tcactgacag cgtgagcccg cggcggctgc tgccatggtg gctggcggcc      60

gggtaagggt ctgagtggat ctcctgccag gccagagcgc cttcgggggc cgcggcggaa     120

ggccaggagt ttgcagccag ggcgccgggt ttgtggtctg cagtgtcgtg aggctgaggt     180

gcagcatgtc tagactggga gccctgggtg gtgcccgtgc cgggctggga ctgttgctgg     240

gtaccgccgc cggccttgga ttcctgtgcc tcctttacag ccagcgatgg aaacggaccc     300

agcgtcatgg ccgcagccag agcctgccca actccctgga ctatacgcag acttcagatc     360

ccggacgcca cgtgatgctc ctgcgggctg tcccaggtgg ggctggagat gcctcagtgc     420

tgcccagcct tccacgggaa ggacaggaga aggtgctgga ccgcctggac tttgtgctga     480

ccagccttgt ggcgctgcgg cgggaggtgg aggagctgag aagcagcctg cgagggcttg     540

cgggggagat tgttggggag gtccgatgcc acatggaaga gaaccagaga gtggctcggc     600

ggcgaaggtt tccgtttgtc cgggagagga gtgactccac tggctccagc tctgtctact     660

tcacggcctc ctcgggagcc acgttcacag atgctgagag tgaagggggt tacacaacag     720

ccaatgcgga gtctgacaat gagcgggact ctgacaaaga aagtgaggac ggggaagatg     780

aagtgagctg tgagactgtg aagatgggga gaaaggattc tcttgacttg gaggaagagg     840

cagcttcagg tgcctccagt gccctggagg ctggaggttc ctcaggcttg gaggatgtgc     900

tgcccctcct gcagcaggcc gacgagctgc acaggggtga tgagcaaggc aagcgggagg     960

gcttccagct gctgctcaac aacaagctgg tgtatggaag ccggcaggac tttctctggc    1020

gcctggcccg agcctacagt gacatgtgtg agctcactga ggaggtgagc gagaagaagt    1080

catatgccct agatggaaaa gaagaagcag aggctgctct ggagaagggg gatgagagtg    1140

ctgactgtca cctgtggtat gcggtgcttt gtggtcagct ggctgagcat gagagcatcc    1200

agaggcgcat ccagagtggc tttagcttca aggagcatgt ggacaaagcc attgctctcc    1260

agccagaaaa ccccatggct cactttcttc ttggcaggtg gtgctatcag gtctctcacc    1320

tgagctggct agaaaaaaaa actgctacag ccttgcttga aagccctctc agtgccactg    1380

tggaagatgc cctccagagc ttcctaaagg ctgaagaact acagccagga ttttccaaag    1440

caggaagggt atatatttcc aagtgctaca gagaactagg gaaaaactct gaagctagat    1500

ggtggatgaa gttggccctg gagctgccag atgtcacgaa ggaggatttg gctatccaga    1560

aggacctgga agaactggaa gtcattttac gagactaacc acgtttcact ggccttcatg    1620

acttgatgcc actatttaag gtgggggggc ggggaggctt ttttccttag accttgctga    1680

gatcaggaaa ccacacaaat ctgtctcctg ggtctgactg ctacccacta ccactcccca    1740

ttagttaatt tattctaacc tctaacctaa tctagaattg gggcagtact catggcttcc 1800

gtttctgttg ttctctccct tgagtaatct cttaaaaaaa tcaagattca cacctgcccc 1860

aggattacac atgggtagag cctgcaagac ctgagacctt ccaattgctg gtgaggtgga 1920

tgaacttcaa agctatagga acaaagcaca taacttgtca ctttaatctt tttcactgac 1980

taataggact cagtacatat agtcttaaga tcatacctta cctaccaagg taaaaagagg 2040

gatcagagtg gcccacagac attgctttct tatcacctat catgtgaatt ctacctgtat 2100

tcctgggctg gaccacttga taacttccag tgtcctggca gcttttggaa tgacagcagt 2160

ggtatggggt ttatgatgct ataaaacaat gtctgaaaag ttgcctagaa tatattttgt 2220

tacaaacttg aaataaacca aatttgatgt t 2251

<210> 36
<211> 1290
<212> DNA
<213> Homo sapiens

<400> 36
tgacagcgtg agcccgcggc ggctgctgcc atggtggctg gcggccgggt aagggtctga 60

gtggatctcc tgccaggcca gagcgccttc gggggccgcg gcggaaggcc aggagtttgc 120

agccagggcg ccgggtttgt ggtctgcagt gtcgtgaggc tgaggtgcag catgtctaga 180

ctgggagccc tgggtggtgc ccgtgccggg ctgggactgt tgctgggact atacgcagac 240

ttcagatccc ggacgccacg tgatgctcct gcgggctgtc ccaggtgggg ctggagatgc 300

ctcagtgctg cccagccttc cacgggaagg acaggagaag gtgctggacc gcctggactt 360

tgtgctgacc agccttgtgg cgctgcggcg ggaggtggag gagctgagaa gcagcctgcg 420

agggcttgcg ggggagattg ttggggaggt ccgatgccac atggaagaga accagagagt 480

ggctcggcgg cgaaggtttc cgtttgtccg ggagaggagt gactccactg gctccagctc 540

tgtctacttc acggcctcct cgggagccac gttcacagat gctgagagtg aagggggtta 600

cacaacagcc aatgcggagt ctgacaatga gcgggactct gacaaagaaa gtgaggacgg 660

ggaagtgaag tgagctgtga gactgtgaag atggggagaa aggattctct tgacttggag 720

gaagaggcag cttcaggtgc ctccagtgcc ctggaggctg gaggttcctc aggcttggag 780

gatgtgctgc ccctcctgca gcaggccgac gagctgcaca ggggtgatga gcaaggcaag 840

cgggagggct tccagctgct gctcaacaac aagctggtgt atggaagccg gcaggacttt 900

ctctggcgcc tggcccgagc ctacagtgac atgtgtgagc tcactgagga ggtgagcgag 960

aagaagtcat atgccctaga tggaaaagaa gaagcagagg ctgctctgga gaagggggat 1020

gagagtgctg actgtcacct gtggtatgcg gtgctttgtg gtcagctggc tgagcatgag 1080

agcatccaga ggcgcatcca gagtggcttt agcttcaagg agcatgtgga caaagccatt 1140

gctctccagc cagaaaaccc catggctcac tttcttcttg gcaggtggtg ctatcaggtc 1200

tctcacctga gctggctaga aaaaaaacta cagccttgct tgaaagccct ctcagtgcca 1260

ctgtggaaga tgccctccag agcttcctaa 1290

## Description

The present invention relates to newly identified proteins as markers for the detection of tumors, or as targets for their treatment, particularly of tumors affecting lung, colon, breast and prostate. Also provided are affinity ligands capable of selectively interacting with the newly identified markers, as well as methods for tumor diagnosis and therapy using such ligands.

### Background of the invention

### Tumor markers (or biomarkers)

Tumor markers are substances that can be produced by tumor cells or by other cells of the body in response to cancer. In particular, a protein biomarker is either a single protein or a panel of different proteins, that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a prophylactic intervention.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For example, prostate-specific antigen (PSA) levels are often used to screen men for prostate cancer, but this is controversial since elevated PSA levels can be caused by both prostate cancer or benign conditions, and most men with elevated PSA levels turn out not to have prostate cancer.

Another tumor marker, Cancer Antigen 125, (CA 125), is sometimes used to screen women who have an increased risk for ovarian cancer. Scientists are studying whether measurement of CA 125, along with other tests and exams, is useful to find ovarian cancer before symptoms develop. So far, CA 125 measurement is not sensitive or specific enough to be used to screen all women for ovarian cancer. Mostly, CA 125 is used to monitor response to treatment and check for recurrence in women with ovarian cancer. Finally, human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are the still the main modality of local treatment of cancer in the majority of patients. However, these treatments are effective only at initial phases of the disease and in particular for solid tumors of epithelial origin, as is the case of colon, lung, breast, prostate and others, while they are not effective for distant recurrence of the disease. In some tumor classes, chemotherapy treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solution that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes, show only a partial efficacy and are frequently associated with chemotherapies to increase their therapeutic effect. Administration of Trastuzumab (Herceptin), a commercial monoclonal antibody targeting HER2, a protein overproduced in about 20% of breast cancers, in conjunction with Taxol adjuvant chemotherapy induces tumor remission in about 42% of the cases. Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (Adams G.P. and Weiner L.M. (2005) Monoclonal antibody therapy cancer. Nat Biotechnol. 23:1147-57).

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Summary of the invention

The present invention provides new means for the detection and treatment of tumors, in particular colo-rectal, lung, prostate and breast cancers, based on the identification of protein markers specific for these tumor types, namely: a) Collectin-11 (COLEC11) protein, b) Follistatin-like protein 5 (FSTL5) and c) FAM82C (FAM82A2) protein.

In preferred embodiments, the invention provides the use of a) COLEC11 protein as a marker or target for colon tumor, b) FSTL5 protein as a marker or target for colon and prostate tumors, c) FAM82A2 as a marker or target for breast, colon and lung tumors.

The invention also provides a method for the diagnosis of these cancer types, comprising a step of detecting the above-identified markers in a biological sample, e.g. in a tissue sample of a subject suspected of having or at risk of developing malignancies or susceptible to cancer recurrences. In particular, the protein markers of the invention allow to specifically detect lung, colon, breast and prostate cancers, according to their tumor-specificity, namely: a) COLEC11 protein for colon tumor; b) FSTL5 protein for colon and prostate tumors; c) FAM82A2 protein for breast, colon and lung tumors.

In addition, the tumor markers identify novel targets for affinity ligands which can be used for therapeutic applications, especially in the treatment of colon, lung, prostate and breast proliferative diseases. Also provided are affinity ligands, particularly antibodies, capable of selectively interacting with the newly identified protein markers.

### Detailed disclosure of the invention

The present invention is based on the surprising finding of antibodies that are able to specifically stain tumor tissues from patients, while negative or very poor staining is observed in normal tissues from the same patients. These antibodies have been found to specifically bind to proteins for which no previous association with tumor has been reported. Hence, in a first aspect, the invention provides a tumor marker which is selected from the group consisting of:
a) Collectin-11 in one of its variant isoforms **SEQ ID NO:1, SEQ ID NO:2 SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12** or a different isoform having sequence identity of at least 80%, preferably at least 90%, more preferably at least 95% to any of SEQ ID NO:1, SEQ ID NO:2 SEQ ID NO:3, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 or SEQ ID NO:12; or a nucleic acid molecule containing a sequence coding for a Collectin-11 protein, said encoding sequence being preferably selected from **SEQ ID NO: 18, SEQ ID NO: 19, SEQ ID NO: 20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30 and SEQ ID NO:31;**
b) FSTL5 protein in one of its variant isoforms **SEQ ID NO:13** or **SEQ ID NO:14,** or a different isoform having sequence identity of at least 80%, preferably at least 90%, more preferably at least 95% to SEQ ID NO:13 or SEQ ID NO:14; or a nucleic acid molecule containing a sequence coding for a FSTL5 protein, said encoding sequence being preferably selected from **SEQ ID NO: 32, SEQ ID NO: 33;**
c) FAM82A2 protein in one of its variant isoforms **SEQ ID NO:15, SEQ ID NO:16 or SEQ ID NO:17**, or a different isoform having sequence identity of at least 80%, preferably at least 90%, more preferably at least 95% to SEQ ID NO:15, SEQ ID NO:16 or SEQ ID NO:17; or a nucleic acid molecule containing a sequence coding for a FAM82A2 protein, said encoding sequence being preferably selected from **SEQ ID NO: 34, SEQ ID NO: 35** and **SEQ ID NO: 36;**

Collectin-11 (Gene names: COLEC11, Gene ID: ENSG00000118004, Protein name: Collectin-11, Alternative name(s): Collectin kidney protein 1, CL-K1, ORF Names: UNQ596/PRO1182, Protein ID: ENSP00000384882, ENSP00000339168, ENSP00000236693, ENSP00000371494, ENSP00000385130, ENSP00000385653, ENSP00000385827, Transcript ID: ENST00000402794, ENST00000349077, ENST00000236693, ENST00000382062, ENST00000403096, ENST00000402922, ENST00000404205) is a protein without previous known association with tumor classes under investigation and is preferably used as a marker for colon tumor and in general for cancers of these types. As described below, an antibody generated towards the COLEC11 protein shows a selective immunoreactivity in histological preparation of colo-rectal cancer tissues, which indicates the presence of COLEC11 in these cancer samples and makes COLEC11 protein and its antibody highly interesting tools for specifically distinguishing a colo-rectal cancer from a normal state.

Follistatin-related protein 5 (Gene name: FSTL5, Synonyms: KIAA1263, Gene ID: ENSG00000168843, Protein name(s): Follistatin-related protein 5, Follistatin-like 5, Protein ID: ENSP00000368462, ENSP00000305334, Transcript ID: ENST00000379164, ENST00000306100) is a protein without previous known association with tumor classes under investigation and is preferably used as a marker for colon and prostate tumors, and in general for cancers of these types. As described below, an antibody generated towards a fragment of FSTL5 shows a selective immunoreactivity in histological preparation of colo-rectal cancer tissues and prostate cancer tissues, which indicates the presence of this protein in these cancer samples.

FAM82A2 (Gene ID: ENSG00000137824, Gene Name: FAM82A2, Synonyms: FAM82C, PTPIP51, Protein name: FAM82A2, Regulator of microtubule dynamics protein 3, RMD-3, hRMD-3, FAM82A2, FAM82C, Protein tyrosine phosphatase-interacting protein 51, TCPTP-interacting protein 51, Cerebral protein 10; Protein ID: ENSP00000260385, ENSP00000342493, ENSP00000380607, Transcript ID: ENST00000260385, ENST00000338376, ENST00000397465) is a protein without previous known association with tumor classes under investigation and is preferably used as a marker for breast, colon and lung tumors, and in general for cancers of these types. As described below, an antibody generated towards a fragment of FAM82A2 shows a selective immunoreactivity in histological preparation of breast cancer tissues, colon cancer tissues and lung cancer tissues, which indicates the presence of this protein in these cancer samples.

A further aspect of this invention is a method of screening a tissue sample for malignancy, which comprises determining the presence in said sample of at least one of the above-mentioned tumor markers. This method includes detecting either the marker protein, e.g. by means of labeled monoclonal or polyclonal antibodies that specifically bind to the target protein, or the respective mRNA, e.g. by means of polymerase chain reaction techniques such as RT-PCR. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

In one embodiment, the invention provides a method of screening a sample of colon or colo-rectal tissue for malignancy, which comprises determining the presence in said sample of a tumor marker selected from COLEC11, FSTL5 and FAM82A2 proteins, variants or isoforms or combinations thereof as described above. In another embodiment, the invention provides a method of screening a sample of lung tissue for malignancy, which comprises determining the presence in said sample of the FAM82C protein tumor marker, variants or isoforms thereof as described above. In a further embodiment, the invention provides a method of screening a sample of breast tissue for malignancy, which comprises determining the presence in said sample of the FAM82C protein tumor marker, variants or isoforms thereof as described above. In a yet further embodiment, the invention provides a method of screening a sample of prostate tissue for malignancy, which comprises determining the presence in said sample of the FSTL5 protein tumor marker, variants or isoforms thereof as described above.

A further aspect of the invention is a method *in vitro* for determining the presence of a tumor in a subject, which comprises the steps of:
(1) providing a sample of the tissue suspected of containing tumor cells;
(2) determining the presence of a tumor marker as above defined, or a combination thereof in said tissue sample by detecting the expression of the marker protein or the presence of the respective mRNA transcript;
wherein the detection of one or more tumor markers in the tissue sample is indicative of the presence of tumor in said subject.

The methods and techniques for carrying out the assay are known to one skilled in the art and are prefarbly based on immunoreactions for detecting proteins and on PCR methods for the detection of mRNAs. The same methods for detecting proteins or mRNAs from a tissue sample as disclosed above can be applied.

A further aspect of this invention is the use of the tumor markers herein provided as targets for the identification of candidate antitumor agents. Accordingly, the invention provides a method for screening a test compound which comprises contacting the cells expressing a tumor-associated protein selected from Collectin-1, Follistatin-like protein 5 and FAM82A2 with the test compound, and determining the binding of said compound to said cells. In addition, the ability of the test compound to modulate the activity of each target molecule can be assayed.

A further aspect of the invention is an antibody or a fragment thereof which is able to specifically recognize and bind to one of the tumor-associated proteins described above. The term "antibody" as used herein refers to all types of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

The antibodies to the tumor markers of the invention can be used to detect the presence of the marker in histologic preparations or to distinguish tumor cells from normal cells. To that purpose, the antibodies may be labeled with radiocative, fluorescent or enzyme labels.

In addition, the antibodies can be used for treating proliferative diseases by modulating, e.g. inhibiting or abolishing the activity of a target protein according to the invention. Therefore, in a further aspect the invention provides the use of antibodies to a tumor-associated protein selected from Collectin-1, Follistatin-like protein 5 and FAM82A2, for the preparation of a therepautic agent for the treatment of proliferative diseases. For use in therapy, the antibodies can be formulated with suitable carriers and excipients, optionally with the addition of adjuvants to enhance their effetcs.

A further aspect of the invention relates to a diagnostic kit containing suitable means for detection, in particular the polypeptides or polynucleotides, antibodies or fragments or derivatives thereof described above, reagents, buffers, solutions and materials needed for setting up and carrying out the immunoassays, nucleic acid hybridization or PCR assays described above. Parts of the kit of the invention can be packaged individually in vials or bottles or in combination in containers or multicontainer units.

### Description of the Figures

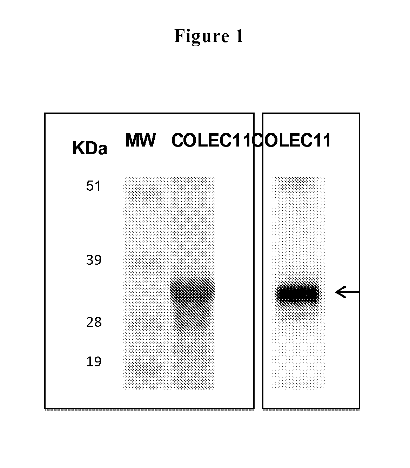

**Figure 1****. Analysis of purifiedCOLEC11recombinant protein expressed in *E.coli***
   Left panel: Comassie staining of purified His-tagCOLEC11fusion protein espressed in *E. coli* separated by SDS-PAGE; Right panel: WB on the purified recombinant COLEC11 protein stained with anti-COLEC11 antibody. Arrow marks the protein band of the expected size.
   Molecular weight markers are reported on the left.
**Figure 2**. Examples of TMA of tumor (left panels) and normal tissue samples (right panels) stained with anti-COLEC11 antibodies. The antibody-stains specifically tumor cells (in dark gray); Boxed image represents a zoomed view of the sample section.
**Figure 3****. Expression of COLEC11 in transiently transfected HeLa cells**
   A)Western blot analysis of COLEC11 expression in total protein extracts from HeLa cells (corresponding to 1x106 cells) transfected with the empty vector pcDNA3 (lane 1) or with the plasmid construct encoding the COLEC11 gene (lane 2) stained with anti-COLEC11 antibody. Arrow marks the expected COLEC11 band. Molecular weight markers are reported on the left. B) Flow cytometry analysis of COLEC11 cell surface localization in HeLa cells transfected with the empty vector pcDNA3 (dashed line) or with the plasmid construct encoding the COLEC11 gene (solid line). X axys, Fluorescence scale; Y axys, Cells (expressed as % relative to major peaks).

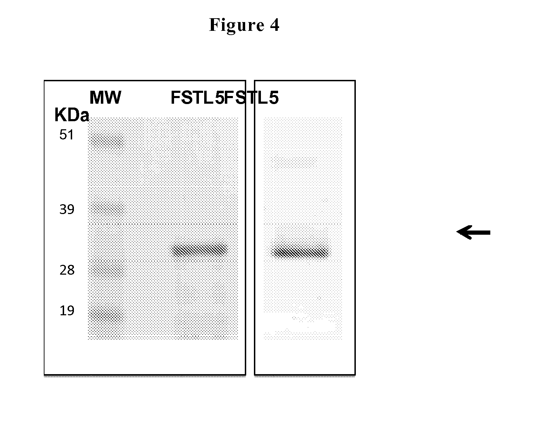

**Figure 4****. Analysis of purified FSTL5 recombinant protein expressed in *E.coli***
   Left panel: Comassie staining of purified His-tag FSTL5 fusion protein espressed in *E. coli* separated by SDS-PAGE; Right panel: WB on the purified recombinant FSTL5protein stained with anti-FSTL5antibody. Arrow marks the protein band of the expected size.
   Molecular weight markers are reported on the left.

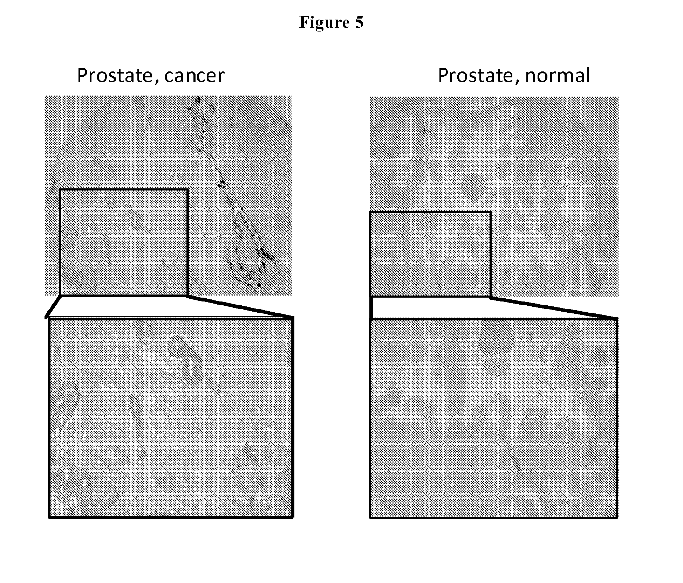

**Figure 5** Examples of TMA of tumor (left panels) and normal tissue samples (right panels) stained with anti-Follistatin-like V antibodies. The antibody-stains specifically tumor cells (in dark gray); Boxed images represent zoomed views of corresponding sample sections

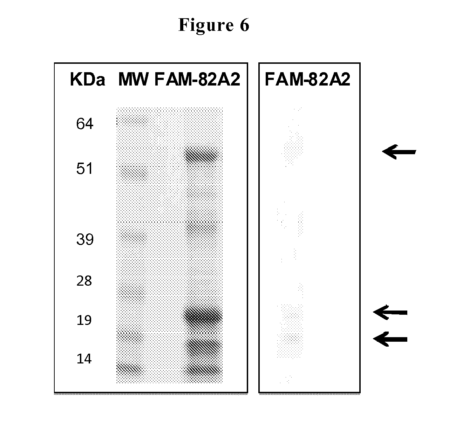

**Figure 6****. Analysis of purified FAM-82A2 recombinant protein expressed in *E.coli***
   Left panel: Comassie staining of purified His-tag FAM-82A2 fusion protein espressed in *E. coli* separated by SDS-PAGE; Right panel: WB on the purified recombinant FAM82A2 protein stained with anti-FAM82A2 antibody. Arrow marks the protein band of the expected size. On the gel appear degradation products verified by mass spectrometry. Molecular weight markers are reported on the left.

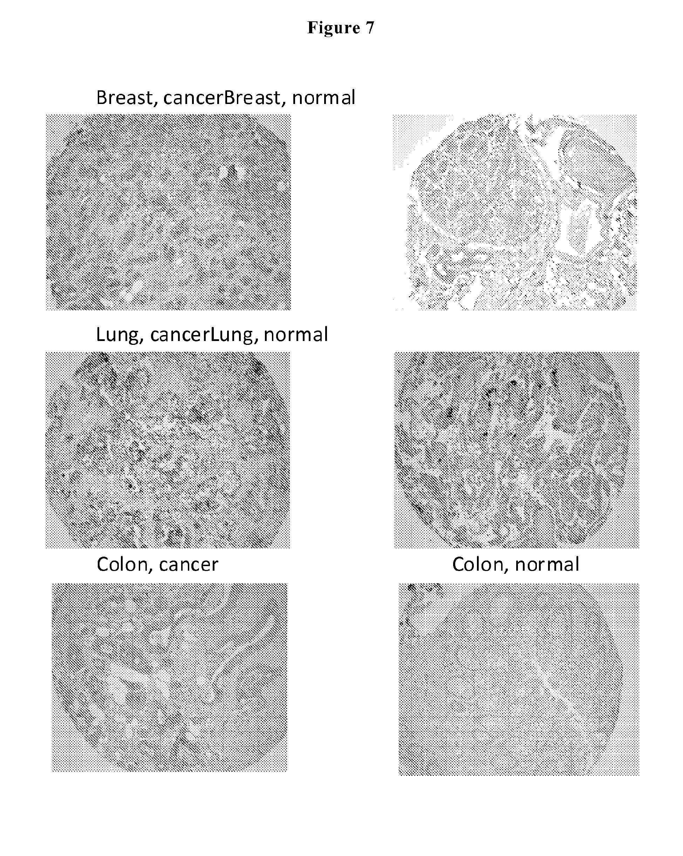

**Figure 7****.** Examples of TMA of tumor (left panels) and normal tissue samples (right panels) stained with anti-FAM82A2 antibodies. The antibodies stain specifically tumor cells (in dark gray)
   The following examples further illustrate the invention.

### EXAMPLES

### Example 1. Generation of recombinant human protein antigens and antibodies to identify tumor markers

### Methods

The entire coding region or suitable fragments of the genes encoding the target proteins, were designed for cloning and expression using bioinformatic tools with the human genome sequence as template (Lindskog M et al (2005). Where present, the leader sequence for secretion was replaced with the ATG codon to drive the expression of the recombinant proteins in the cytoplasm of E. coli. For cloning, genes were PCR-amplified from cDNA derived from Mammalian Gene Collection (http://mgc.nci.nih.gov/) clones using specific primers so as to fuse a 6 histidine tag sequence at the 3' end, annealed to in house developed vectors, derivatives of vector pSP73 (Promega) adapted for the T4 ligation independent cloning method (Nucleic Acids Res. 1990 October 25; 18(20): 6069-6074) and used to transform *E. coli* NovaBlue cells recipient strain. *E. coli* tranformants were plated onto selective LB plates containing 100 µg/ml ampicillin (LB Amp) and positive E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (Studier, 2005) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4 °C. All proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations.

To generate antisera, the purified proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were successfully isolated by PCR from specific clones of the Mammalian Gene Collection using primers specific for each gene. In particular, for the COLEC11 gene, a fragment corresponding to nucleotides 316-1053 of the transcript (SEQ ID ENST00000349077) of and encoding an amino acid region from 26 to 271 (SEQ ID ENSP00000339168) was obtained.

For the FSTL5 gene, a fragment corresponding to nucleotides 503-1225 of the transcript (SEQ ID ENST00000306100) of and encoding an amino acid region from 23 to 263 (SEQ ID ENSP00000305334) was obtained.

For the FAM82A2, a fragment corresponding to nucleotides 94-1410 of the transcript (SEQ ID ENST00000260385) of and encoding an amino acid region from 32 to 470 (SEQ ID ENSP00000260385) was obtained.

A clone encoding the correct amino acid sequence was identified for each gene/gene fragment and, upon expression in *E. coli,* a protein of the correct size was produced and subsequently purified using affinity chromatography (Figures 1,4,6 left panel). Antibodies generated by immunization specifically recognized their target proteins in Western blot (WB) (Figures 1, 4,6, right panel).

### Example 2. Tissue profiling by immune-histochemistry

### Methods

The analysis of the antibodies' capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

A tissue microarray was prepared containing 100 formalin-fixed paraffin-embedded cores of human tissues from patients affected by colorectal cancer, ovarian cancer, breast cancer, lung cancer, prostate cancer and corresponding normal tissues and analyzed using the specific antibody sample. Briefly, each TMA slide included tumor tissue samples representative of different well pedigreed patients, representing the 5 cancer types, and an equal number of normal tissue samples from the same patients as controls. In total, the TMA design consisted in 10 tumor samples per each tumor class and 10 normal tissue from 5 well pedigreed patients (equal to two tumor samples and 2 normal tissues from each patient) to identify promising target molecules differentially expressed in cancer and normal tissues. The direct comparison between tumor and normal tissues of each patient allowed the identification of antibodies that stain tumor cells and provide indication of target expression in the tumor under investigation.

All formalin fixed, paraffin embedded tissues used as donor blocks for TMA production were selected from the archives at the IEO (Istituto Europeo Oncologico, Milan). Corresponding whole tissue sections were examined to confirm diagnosis and tumour classification, and to select representative areas in donor blocks. Normal tissues were defined as microscopically normal (non-neoplastic) and were generally selected from specimens collected from the vicinity of surgically removed tumors. The TMA production was performed essentially as previously described (Kononen J et al (1998) Nature Med. 4:844-847; Kallioniemi OP et a/ (2001) Hum. MoI. Genet. 10:657-662). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500" della dita BioRep (Milan) until a complete TMA design was produced. TMA recipient blocks were baked at 42 <0>C for 2 h prior to sectioning. The TMA blocks were sectioned with 2-3 µm thicknes using a waterfall microtome (Leica), and placed onto poli-L-lysinated glass slides for immunohistochemical analysis. Automated immunohistochemistry was performed as previously described (Kampf C. et al (2004) Clin. Proteomics 1:285-300). In brief, the glass slides were incubated for 30' min in 60 °C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ^{™} dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma-Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

A TMA design was obtained, representing tumor tissue samples from 5 tumor classes (lung, ovary, prostate, breast and colon) and normal tissues, derived from 5 patients for each tumor type. The results from tissue profiling showed that the antibodies specific for the recombinant proteins (see Example 1) are strongly immunoreactive on several cancer tissues, indicating the presence of the target proteins in tumors tissues, while no or poor reactivity was detected in normal tissues. Based on this finding, the detection of target proteins in tissue samples can be associated with the specific tumor/s.

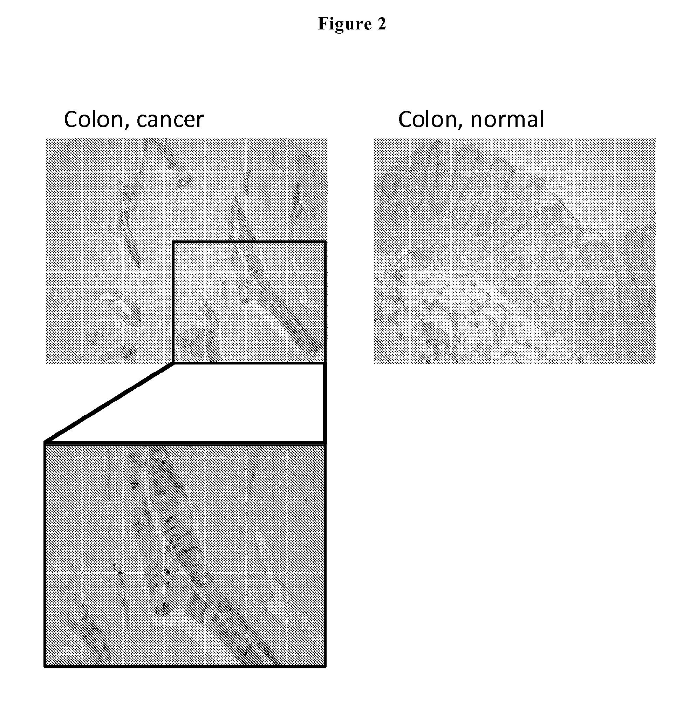

The capability of target-specific antibodies to stain different tumor tissues is summarized in Table. Representative examples of microscopic enlargements of tissue samples stained by each antibody are reported in Figures 2, 5, 7.

**TABLE: TUMOR MARKERS IDENTIFIED BY TMA**

| ***TARGET*** | ***TISSUE MICROARRAY*** | | | | |
|---|---|---|---|---|---|
| | ***BREAST*** | ***COLON*** | ***LUNG*** | ***OVARY*** | ***PROSTATE*** |
| ***COLLEC 11*** | ***0*/*5*** | ***2*/*5*** | ***0*/*5*** | ***0*/*5*** | ***0*/*5*** |
| ***FSTL 5*** | ***0*/*5*** | ***1*/*5*** | ***0*/*5*** | ***0*/*5*** | **1/5** |
| ***FAM82A2*** | ***3*/*5*** | ***3*/*5*** | ***3*/*5*** | ***0*/*5*** | ***0*/*5*** |

The table reports the number of positive tumor samples out of the five screened after staining with the target specific antibodies.

### Example 3. Expression and cell localization of target protein in transfected mammalian cells

### Methods

The specificity of the antibodies for each target proteins was assessed by Western blot analysis on total protein extracts from eukaryotic cells transiently transfected with plasmid constructs containing the complete sequences of the genes encoding the target proteins.

To this aim, cDNA were generated from pools of total RNA derived from Human testis, Human placenta, Human bone marrow, Human fetal brain, in reverse transcription reactions and the entire coding regions (ORF) were PCR-amplified with specific primers pairs. PCR products were cloned into plasmid pcDNA3 (Invitrogen). HeLa cells were grown in DMEM-10% FCS supplemented with 1 mM Glutamine were transiently transfected with preparation of the resulting plasmids and with the empty vector as negative control using the Lipofectamine-2000 transfection reagent (Invitrogen). After 48 hours, cells were collected, lysed with PBS buffer containing 1% Triton X100 and expression of target protein(s) was assessed by Western blot analysis on total cell extracts (corresponding to 1x10⁶ cells) using specific antibodies . Western blot was performed by separation of the protein extracts on pre-cast SDS-PAGE gradient gels (NuPage 4-12 % Bis-Tris gel, Invitrogen) under reducing conditions, followed by electro-transfer to nitrocellulose membranes (Invitrogen) according to the manufacturer's recommendations. The membranes were blocked in blocking buffer composed of 1x PBS-0.1% Tween 20 (PBST) added with 10 % dry milk, for 1 h at room temperature, incubated with the antibody diluted 1:2500 in blocking buffer containing 1% dry milk and washed in PBST-1%. The secondary HRP-conjugated antibody (goat anti-mouse immunoglobulin-HRP, Perkin Elmer) was diluted 1:5000 in blocking buffer and chemiluminescence detection was carried out using a Chemidoc-IT UVP CCD camera (UVP) and the Western lightning^{Tm} cheminulescence Reagent Plus (Perkin Elmer), according to the manufacturer's protocol.

Surface localization of target proteins was assessed in HeLa transfected cells by cell surface staining and Flow Cytometry (FACS) analysis. HeLa cells transfected with the Colec-11 construct or with the empty vector (2x10⁴ per well) were pelletted in 96 U-bottom microplates by centrifugation at 200 x g for 5 min at 4°C and incubated for 1 hour at 4°C with the appropriate dilutions of antibodies against the target proteins. The cells were washed twice in PBS-5%FCS and incubated for 20 min with the appropriate dilution of R-Phycoerythrin (PE)-conjugated secondary antibodies (Jackson Immuno Research, PA, USA) at 4°C. After washing, bacteria were analysed by a FACS Canto II flow cytometer (Becton Dickinson). Data were analyzed with FlowJo 8.3.3 program.

### Results

To confirm the antibody specificity, the complete coding sequence/s for each target protein were cloned in a eukaryotic expression vector and used for transient transfection of HeLa cells.

Expression of target proteins was detected by Western blot in total protein extracts from HeLa cells transfected with the constructs encoding for the target proteins using their specific antibodies. A band of the expected size was visible in HeLa cells transfected with plasmid expressing COLEC11, while the same band was not visible in HeLa cells transfected with the empty pcDNA3 plasmid. The antibody recognized specifically its target protein, since almost a unique single protein band was detected.

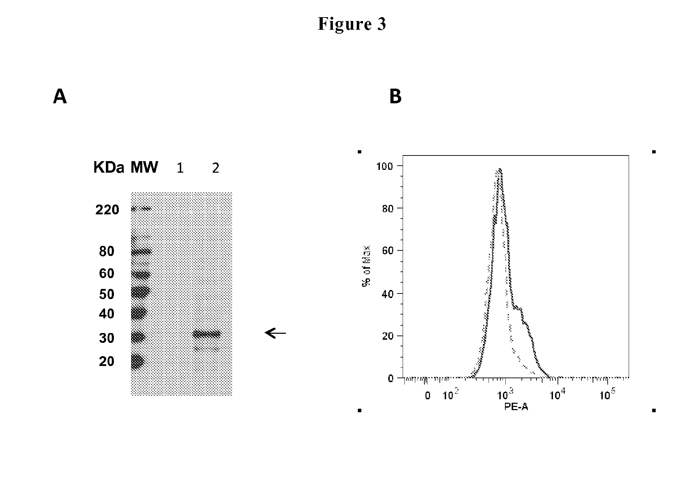

Surface localization of target proteins was addressed by FACS analysis of transiently transfected cells stained with the specific antibodies. COLEC11 is predicted to be a secreted protein and FACS analysis of cells transfected with the construct encoding COLEC11 is consistent with this information; specific COLEC11 antibodies showed a limited binding on the surface of transfected cells, while no binding was observed on cells transfected with the empty pcDNA3 vector. This indicates that a minor fraction of COLEC11 target proteins are localized on the cell surface and are accessible to the external environment. Results are represented in Figure 3.

## Claims

1. A tumor marker which is selected from the group consisting of:
a) Collectin-11 protein in one of its variant isoforms **SEQ ID NO:1, SEQ ID NO:2 SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11** or **SEQ ID NO:12**, or a different isoform having sequence identity of at least 80%, preferably at least 90%, more preferably at least 95% to any of SEQ ID NO:1, SEQ ID NO:2 SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 or SEQ ID NO:12; or a nucleic acid molecule containing a sequence coding for a Collectin-11 protein, said encoding sequence being preferably selected from **SEQ ID NO: 18, SEQ ID NO: 19, SEQ ID NO: 20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30** and **SEQ ID NO:31;**
b) FSTL5 protein in one of its variant isoforms **SEQ ID NO:13** or **SEQ ID NO:14,** or a different isoform having sequence identity of at least 80%, preferably at least 90%, more preferably at least 95% to SEQ ID NO:13 or SEQ ID NO:14; or a nucleic acid molecule containing a sequence coding for a FSTL5 protein, said encoding sequence being preferably selected from **SEQ ID NO: 32** and **SEQ ID NO: 33**;
c) FAM82A2 protein in one of its variant isoforms **SEQ ID NO:15, SEQ ID NO:16** or **SEQ ID NO:17,** or a different isoform having sequence identity of at least 80%, preferably at least 90%, more preferably at least 95% to SEQ ID NO:15, SEQ ID NO:16 or SEQ ID NO:17; or a nucleic acid molecule containing a sequence coding for a FAM82A2 protein, said encoding sequence being preferably selected from **SEQ ID NO: 34, SEQ ID NO: 35** and **SEQ ID NO: 36.**

2. A tumor marker according to claim 1, for use in the detection of lung or breast cancer, wherein said tumor marker is FAM82A2.

3. A tumor marker according to claim 1, for use in the detection of colon or colo-rectal cancers, wherein said tumor marker is selected from COLEC11 protein and FSTL5, or a combination thereof.

4. A tumor marker according to claim 1, for use in the detection of prostate cancer, wherein said tumor marker is FSTL5.

5. A method of screening a tissue sample for malignancy, said method comprising determining the presence in said sample of at least one of the above-mentioned tumor markers.

6. A method according to claim 5, wherein the tissue sample is a sample of colon or colo-rectal tissue, said method comprising determining the presence in said sample of a tumor marker selected from COLEC11 protein and FSTL5, or a combination thereof.

7. A method according to claim 5, wherein the tissue sample is a sample of lung tissue, said method comprising determining the presence in said sample of the FAM82A2 tumor marker.

8. A method according to claim 5, wherein the tissue sample is a sample of breast tissue, said method comprising determining the presence in said sample of the FAM82A2 tumor marker.

9. A method according to claim 5, wherein the tissue sample is a sample of prostate tissue, said method comprising determining the presence in said sample of the FSTL5 tumor marker.

10. A method according to claim 5, wherein the tumor marker is a protein, said method being based on immunoradiometric, immunoenzymatic or immunohistochemical techniques.

11. A method according to claim 5, wherein the tumor marker is a nucleic acid molecule, said method being based on polymerase chain reaction techniques.

12. A method *in vitro* for determining the presence of a tumor in a subject, which comprises the steps of:
(1) providing a sample of the tissue suspected of containing tumor cells;
(2) determining the presence of a tumor marker according to claim 1 or a combination thereof as per claims 2-4 in said tissue sample by detecting the expression of the marker protein or the presence of the respective mRNA transcript;
wherein the detection of one or more tumor markers in the tissue sample is indicative of the presence of tumor in said subject.

13. A method of screening a test compound as an antitumor candidate, which comprises contacting cells expressing a tumor marker protein according to claim 1 with the test compound, and determining the binding of said compound to said cells.

14. An antibody or a fragment thereof which is able to specifically recognize and bind to one of the tumor marker proteins according to claim 1.

15. An antibody according to claim 14, which is either monoclonal or polyclonal.

16. The use of an antibody according to claim 14 or 15, for the preparation of a therepautic agent for the treatment of proliferative diseases.

17. A diagnostic kit containing an antibody according to claims 14-15 and/or an oligonucleotide complementary to a nucleic acid molecule encoding a tumor marker according to claim 1, and optionally reagents, buffers, solutions and materials to carry out an immunoassay or a PCR assay.
